# EUROPEAN PATENT APPLICATION

(11) **EP 4 035 680 A1**
(43) Date of publication of application: **03.08.2022**
(21) Application number: 20877196.4
(22) Date of filing: 16.10.2020
(51) Int. Cl.: A61K 39/395, A61K 45/00, A61P 1/04, A61P 1/14, A61P 1/16, A61P 3/10, A61P 13/12, A61P 19/00, A61P 27/02, A61P 17/10, A61K 38/16

(54) **THERAPY FOR DIABETES USING STEM CELL MIGRATION AGENT**

(30) Priority: 18.10.2019 JP 2019191369
(71) Applicant: Biozipcode, Inc., Otsu-shi, Shiga 520-2192 (JP)
(72) Inventor: KOJIMA, Hideto, Otsu-shi, Shiga 520-2192 (JP); TERASHIMA, Tomoya, Otsu-shi, Shiga 520-2192 (JP); KATAGI, Miwako, Otsu-shi, Shiga 520-2192 (JP)
(74) Representative: Engelhard, Markus
(86) International application number: PCT/JP2020/039045
(87) International publication number: WO 2021/075536

(57) **Abstract**

The present disclosure provides a therapy for diabetes that targets abnormal stem cells in combination with stem cell migration. In one embodiment, the present disclosure provides a therapy for diabetes and/or diabetes-related diseases and disorders and/or symptoms that targets abnormal stem cells in combination with stem cell migration. In one embodiment, the present disclosure provides diagnosis of diabetes and/or diabetes-related diseases and disorders and/or symptoms, or the risk thereof, using abnormal stem cell migration and/or residence as an indicator.

## Description

### [Technical Field]

The present disclosure relates to therapy of diabetes mellitus and/or an associated disease thereof with a combination of migration of stem cells and suppression of abnormal stem cells and diagnosis of diabetes mellitus and/or an associated disease thereof using the migration and/or residual state of stem cells as an indicator. More specifically, the present disclosure relates to therapy of diabetes mellitus and/or an associated disease thereof by migration and suppression of an abnormal hematopoietic stem cell and diagnosis of diabetes mellitus and/or an associated disease thereof by detection of the migration of an abnormal hematopoietic stem cell from a specific niche and/or the residual state of an abnormal hematopoietic stem cell in a specific niche.

### [Background Art]

Diabetes mellitus is generally classified into type 1 and type 2 (Non Patent Literature 1). However, since details of the onset of both types are unknown, classification into type 1 and type 2 itself should be considered as lacking validity as classification based on the cause of the diseases. Currently, while various drugs have been developed for therapy of diabetes mellitus, many of those drugs are intended for blood glucose control, which can be effective for prevention of progression of diabetes mellitus but can be insufficient for healing of diabetes mellitus. Moreover, diabetic complications such as neuropathy, nephropathy, hepatopathy, retinopathy, fatty liver, gastrointestinal disorder, delayed bone fracture healing, eating disorder, and dermatopathy also develop as common complications of diabetes mellitus classified into type 1 and type 2. These complications can be difficult to heal once they develop.

### [Citation List]

### [Non Patent Literature]

[NPL 1] Japanese Clinical Practice Guideline for Diabetes 2016, the Japan Diabetes Society, Nankodo Co., Ltd.

### [Summary of Invention]

### [Solution to Problem]

The present inventors have found that therapeutic strategies for diabetes mellitus and/or an associated disease thereof by suppressing abnormal stem cells are more effective when combined with stem cell migration. Based on the new finding, the present disclosure provides a means for therapy and diagnosis of diabetes mellitus and/or an associated disease thereof.

Thus, the present disclosure provides the following.

### (Item 1)

A composition for use in treating and/or preventing diabetes mellitus and/or a disease, disorder, and/or symptom associated with diabetes mellitus, comprising a suppressing agent for an abnormal hematopoietic stem cell (HSC), characterized in that the suppressing agent is administered in combination with a stem cell migration agent.

### (Item 2)

The composition of any of the preceding items, wherein the abnormal HSC is a cell in which a gene or protein selected from the group consisting of CD106 and a functional equivalent thereof is not expressed and/or does not function at a normal level.

### (Item 3)

The composition of any of the preceding items, wherein the expression which is not at a normal level is overexpression.

### (Item 4)

The composition of any of the preceding items, wherein the suppressing agent comprises at least one selected from the group consisting of an anti-CD106 antibody or a functional variant thereof.

### (Item 5)

The composition of any of the preceding items, wherein the abnormal HSC is a cell in which a gene or protein selected from the group consisting of tumor necrosis factor alpha (TNF-α) and proinsulin is not expressed at a normal level.

### (Item 6)

The composition of any of the preceding items, wherein the suppressing agent comprises at least one selected from the group consisting of an anti-TNF-α antibody or a functional variant thereof.

### (Item 7)

The composition of any of the preceding items, wherein the disease, disorder, and/or symptom comprises a diabetic complication.

### (Item 8)

The composition of any of the preceding items, wherein the disease, disorder, and/or symptom is selected from the group consisting of neuropathy, nephropathy, hepatopathy, retinopathy, fatty liver, gastrointestinal disorder, delayed bone fracture healing, eating disorder, and dermatopathy.

### (Item 9)

The composition of any of the preceding items, wherein the stem cell migration agent has an ability to cause the abnormal HSC to migrate from a niche.

### (Item 10)

The composition of any of the preceding items, wherein the stem cell migration agent comprises at least one agent selected from the group consisting of a CXCR4 antagonizing agent, a CXCR2 stimulating agent, an epidermal growth factor receptor (EGFR) inhibiting agent, and a granulocyte colony stimulating factor (G-CSF) agent.

### (Item 11)

The composition of any of the preceding items, wherein the stem cell migration agent comprises at least one selected from the group consisting of Plerixafor, GROβ2 (MIP2), Gefitinib, Erlotinib, Afatinib, Osimertinib, Filgrastim, Nartograstim, Lenograstim, and Pegfilgrastim.

### (Item 12)

A medicament for use in treating and/or preventing diabetes mellitus and/or a disease, disorder, and/or symptom associated with diabetes mellitus, the medicament comprising a combination of a suppressing agent for an abnormal hematopoietic stem cell (HSC) and a stem cell migration agent.

### (Item 13)

A composition for use in treating and/or preventing diabetes mellitus and/or a disease, disorder, and/or symptom associated with diabetes mellitus, the composition comprising a stem cell migration agent, characterized in that the stem cell migration agent is administered in combination with a suppressing agent for an abnormal hematopoietic stem cell (HSC).

### (Item 14)

A composition for use in selecting a treatment for treating and/or preventing diabetes mellitus and/or a disease, disorder, and/or symptom associated with diabetes mellitus, the composition comprising an agent that detects a migration and/or residual state of an abnormal hematopoietic stem cell (HSC).

### (Item 15)

The composition any of the preceding items, wherein the migration and/or residual state is a migration from a niche of a bone marrow and/or a residual state at the niche of the bone marrow.

### (Item 16)

The composition of any of the preceding items, wherein the migration detecting agent comprises a detection agent for CD106 or a functional equivalent thereof.

### (Item 17)

A method for treating and/or preventing diabetes mellitus or a disease, disorder, and/or symptom associated with diabetes mellitus in a subject, the method comprising: administering an effective amount of an agent that reduces or eliminates an abnormal hematopoietic stem cell (HSC) and a stem cell migration agent to the subject.

### (Item 18)

A method for using a migration and/or residual state of an abnormal hematopoietic stem cell (HSC) as an indicator of treatment for treating and/or preventing diabetes mellitus or diabetes mellitus and/or a disease, disorder, and/or symptom associated with diabetes mellitus in a subject, the method comprising: detecting the migration and/or residual state of the abnormal hematopoietic stem cell (HSC) in the subject.

### (Item 19)

The composition of any of the preceding items, wherein the composition is a pharmaceutical composition.

### (Item 20)

The composition of any of the preceding items, further comprising a pharmaceutically acceptable excipient.

### (Item A1)

A method for treating and/or preventing diabetes mellitus and/or a disease, disorder, and/or symptom associated with diabetes mellitus in a subject, the method comprising: administering an effective amount of a suppressing agent for an abnormal hematopoietic stem cell (HSC) and a stem cell migration agent to the subject.

### (Item A2)

The method of any of the preceding items, wherein the abnormal HSC is a cell in which a gene or protein selected from the group consisting of CD106 and a functional equivalent thereof is not expressed and/or does not function at a normal level.

### (Item A3)

The method of item A2, wherein the expression which is not at a normal level is overexpression.

### (Item A4)

The method of any of the preceding items, wherein the suppressing agent comprises at least one selected from the group consisting of an anti-CD106 antibody or a functional variant thereof.

### (Item A5)

The method of any of the preceding items, wherein the abnormal HSC is a cell in which a gene or protein selected from the group consisting of tumor necrosis factor alpha (TNF-α) and proinsulin is not expressed at a normal level.

### (Item A6)

The method of any of the preceding items, wherein the suppressing agent comprises at least one selected from the group consisting of an anti-TNF-α antibody or a functional variant thereof.

### (Item A7)

The method of any of the preceding items, wherein the disease, disorder, and/or symptom comprises a diabetic complication.

### (Item A8)

The method of any of the preceding items, wherein the disease, disorder, and/or symptom is selected from the group consisting of neuropathy, nephropathy, hepatopathy, retinopathy, fatty liver, gastrointestinal disorder, delayed bone fracture healing, eating disorder, and dermatopathy.

### (Item A9)

The method of any of the preceding items, wherein the stem cell migration agent has an ability to cause the abnormal HSC to migrate from a niche.

### (Item A10)

The method of any of the preceding items, wherein the stem cell migration agent comprises at least one agent selected from the group consisting of a CXCR4 antagonizing agent, a CXCR2 stimulating agent, an epidermal growth factor receptor (EGFR) inhibiting agent, and a granulocyte colony stimulating factor (G-CSF) agent.

### (Item A11)

The composition of any of the preceding items, wherein the stem cell migration agent comprises at least one selected from the group consisting of Plerixafor, GROβ2 (MIP2), Gefitinib, Erlotinib, Afatinib, Osimertinib, Filgrastim, Nartograstim, Lenograstim, and Pegfilgrastim.

### (Item A12)

A method for selecting a treatment for treating and/or preventing diabetes mellitus and/or a disease, disorder, and/or symptom associated with diabetes mellitus in a subject, the method comprising: administering an effective amount of an agent that detects a migration and/or residual state of an abnormal hematopoietic stem cell (HSC) to the subject.

### (Item A13)

The method of any of the preceding items, wherein the migration and/or residual state is a migration from a niche of a bone marrow and/or a residual state at the niche of the bone marrow.

### (Item A14)

The method of any of the preceding items, wherein the migration detecting agent comprises a detection agent for CD106 or a functional equivalent thereof.

### (Item A15)

A method for diagnosing a migration and/or residual state of an abnormal hematopoietic stem cell (HSC), or diabetes mellitus or diabetes mellitus and/or a disease, disorder, and/or symptom associated with diabetes mellitus in a subject, the method comprising: detecting the migration and/or residual state of the abnormal hematopoietic stem cell (HSC) in the subject.

### (Item B1)

A composition for use in suppression of an abnormal hematopoietic stem cell (HSC), for use in treating and/or preventing diabetes mellitus and/or a disease, disorder, and/or symptom associated with diabetes mellitus, and for use in combination with a stem cell migration agent.

### (Item B2)

The composition of any of the preceding items, wherein the abnormal HSC is a cell in which a gene or protein selected from the group consisting of CD106 and a functional equivalent thereof is not expressed and/or does not function at a normal level.

### (Item B3)

The composition of any of the preceding items, wherein the expression which is not at a normal level is overexpression.

### (Item B4)

The suppressing agent of any of the preceding items, comprising at least one selected from the group consisting of an anti-CD106 antibody or a functional variant thereof.

### (Item B5)

The composition of any of the preceding items, wherein the abnormal HSC is a cell in which a gene or protein selected from the group consisting of CD34, tumor necrosis factor alpha (TNF-α), and proinsulin is not expressed at a normal level.

### (Item B6)

The composition of any of the preceding items, comprising at least one selected from the group consisting of an anti-TNF-α antibody or a functional variant thereof.

### (Item B7)

The composition of any of the preceding items, wherein the disease, disorder, and/or symptom comprises a diabetic complication.

### (Item B8)

The composition of any of the preceding items, wherein the disease, disorder, and/or symptom is selected from the group consisting of neuropathy, nephropathy, hepatopathy, retinopathy, fatty liver, gastrointestinal disorder, delayed bone fracture healing, eating disorder, and dermatopathy.

### (Item B9)

The composition of any of the preceding items, wherein the stem cell migration agent has an ability to cause the abnormal HSC to migrate from a niche.

### (Item B10)

The composition of any of the preceding items, wherein the stem cell migration agent comprises at least one agent selected from the group consisting of a CXCR4 antagonizing agent, a CXCR2 stimulating agent, an epidermal growth factor receptor (EGFR) inhibiting agent, and a granulocyte colony stimulating factor (G-CSF) agent.

### (Item B11)

The composition of any of the preceding items, wherein the stem cell migration agent comprises at least one selected from the group consisting of Plerixafor, GROβ2 (MIP2), Gefitinib, Erlotinib, Afatinib, Osimertinib, Filgrastim, Nartograstim, Lenograstim, and Pegfilgrastim.

### (Item B13)

A composition for stem cell migration, for use in treating and/or preventing diabetes mellitus and/or a disease, disorder, and/or symptom associated with diabetes mellitus, and for use in combination with a suppressing agent for an abnormal hematopoietic stem cell (HSC).

### (Item B14)

A composition for use in detection of a migration and/or residual state of an abnormal hematopoietic stem cell (HSC) for use in selection of a treatment for treating and/or preventing diabetes mellitus and/or a disease, disorder, and/or symptom associated with diabetes mellitus.

### (Item B15)

The composition of any of the preceding items, wherein the migration and/or residual state is a migration from a niche of a bone marrow and/or a residual state at the niche of the bone marrow.

### (Item B16)

The composition of any of the preceding items, comprising a detection agent for CD106 or a functional equivalent thereof.

### (Item B17)

The composition of any of the preceding items, wherein the composition is a pharmaceutical composition.

### (Item B18)

The composition of any of the preceding items, further comprising a pharmaceutically acceptable excipient.

### (Item C1)

Use of a suppressing agent for an abnormal hematopoietic stem cell (HSC) and a stem cell migration agent in the manufacture of a medicament for treating and/or preventing diabetes mellitus and/or a disease, disorder, and/or symptom associated with diabetes mellitus.

### (Item C2)

The use of any of the preceding items, wherein the abnormal HSC is a cell in which a gene or protein selected from the group consisting of CD106 and a functional equivalent thereof is not expressed and/or does not function at a normal level.

### (Item C3)

The use of any of the preceding items, wherein the expression which is not at a normal level is overexpression.

### (Item C4)

The use of any of the preceding items, wherein the suppressing agent comprises at least one selected from the group consisting of an anti-CD106 antibody or a functional variant thereof.

### (Item C5)

The use of any of the preceding items, wherein the abnormal HSC is a cell in which a gene or protein selected from the group consisting of CD34, tumor necrosis factor alpha (TNF-α), and proinsulin is not expressed at a normal level.

### (Item C6)

The use of any of the preceding items, wherein the suppressing agent comprises at least one selected from the group consisting of an anti-TNF-α antibody or a functional variant thereof.

### (Item C7)

The use of any of the preceding items, wherein the disease, disorder, and/or symptom comprises a diabetic complication.

### (Item C8)

The use of any of the preceding items, wherein the disease, disorder, and/or symptom is selected from the group consisting of neuropathy, nephropathy, hepatopathy, retinopathy, fatty liver, gastrointestinal disorder, delayed bone fracture healing, eating disorder, and dermatopathy.

### (Item C9)

The use of any of the preceding items, wherein the stem cell migration agent has an ability to cause the abnormal HSC to migrate from a niche.

### (Item C10)

The use of any of the preceding items, wherein the stem cell migration agent comprises at least one agent selected from the group consisting of a CXCR4 antagonizing agent, a CXCR2 stimulating agent, an epidermal growth factor receptor (EGFR) inhibiting agent, and a granulocyte colony stimulating factor (G-CSF) agent.

### (Item C11)

The use of any of the preceding items, wherein the stem cell migration agent comprises at least one selected from the group consisting of Plerixafor, GROβ2 (MIP2), Gefitinib, Erlotinib, Afatinib, Osimertinib, Filgrastim, Nartograstim, Lenograstim, and Pegfilgrastim.

### (Item C14)

Use of an agent for detecting a migration and/or residual state of an abnormal hematopoietic stem cell (HSC), in the manufacture of a medicament for use in selection of a treatment for treating and/or preventing diabetes mellitus and/or a disease, disorder, and/or symptom associated with diabetes mellitus.

### (Item C15)

The use of any of the preceding items, wherein the migration and/or residual state is a migration from a niche of a bone marrow and/or a residual state at the niche of the bone marrow.

### (Item C16)

The use of any of the preceding items, wherein the migration detecting agent comprises a detection agent for CD106 or a functional equivalent thereof.

### (Item D1)

A composition for use in treating and/or preventing diabetes mellitus and/or a disease, disorder, and/or symptom associated with diabetes mellitus, comprising a suppressing agent for an abnormal hematopoietic stem cell (HSC), characterized in that the suppressing agent is administered in combination with a stem cell migration agent.

### (Item D2)

The composition of any of the preceding items, wherein the abnormal HSC is a cell in which a gene or protein selected from the group consisting of CD106 and a functional equivalent thereof is not expressed and/or does not function at a normal level.

### (Item D3)

The composition of any of the preceding items, wherein the expression which is not at a normal level is overexpression.

### (Item D4)

The composition of any of the preceding items, wherein the suppressing agent comprises at least one selected from the group consisting of an anti-CD106 antibody or a functional variant thereof.

### (Item D5)

The composition of any of the preceding items, wherein the abnormal HSC is a cell in which a gene or protein selected from the group consisting of tumor necrosis factor alpha (TNF-α), histone deacetylase (HDAC), and proinsulin is not expressed at a normal level.

### (Item D6)

The composition of any of the preceding items, wherein the suppressing agent comprises at least one selected from the group consisting of an anti-TNF-α antibody or a functional variant thereof and an HDAC inhibiting agent (e.g., trichostatin A).

### (Item D7)

The composition of any of the preceding items, wherein the disease, disorder, and/or symptom comprises a diabetic complication.

### (Item D8)

The composition of any of the preceding items, wherein the disease, disorder, and/or symptom is selected from the group consisting of neuropathy, nephropathy, hepatopathy, retinopathy, fatty liver, gastrointestinal disorder, delayed bone fracture healing, eating disorder, and dermatopathy.

### (Item D9)

The composition of any of the preceding items, wherein the stem cell migration agent has an ability to cause the abnormal HSC to migrate from a niche.

### (Item D10)

The composition of any of the preceding items, wherein the stem cell migration agent comprises at least one agent selected from the group consisting of a CXCR4 antagonizing agent, a CXCR2 stimulating agent, an epidermal growth factor receptor (EGFR) inhibiting agent, and a granulocyte colony stimulating factor (G-CSF) agent.

### (Item D11)

The composition of any of the preceding items, wherein the stem cell migration agent comprises at least one selected from the group consisting of Plerixafor, GROβ2 (MIP2), Gefitinib, Erlotinib, Afatinib, Osimertinib, Filgrastim, Nartograstim, Lenograstim, and Pegfilgrastim.

### (Item D12)

A medicament for use in treating and/or preventing diabetes mellitus and/or a disease, disorder, and/or symptom associated with diabetes mellitus, the medicament comprising a combination of a suppressing agent for an abnormal hematopoietic stem cell (HSC) and a stem cell migration agent.

### (Item D13)

A composition for use in treating and/or preventing diabetes mellitus and/or a disease, disorder, and/or symptom associated with diabetes mellitus, the composition comprising a stem cell migration agent, characterized in that the stem cell migration agent is administered in combination with a suppressing agent for an abnormal hematopoietic stem cell (HSC).

### (Item D14)

A composition for use in selecting a treatment for treating and/or preventing diabetes mellitus and/or a disease, disorder, and/or symptom associated with diabetes mellitus, the composition comprising an agent that detects a migration and/or residual state of an abnormal hematopoietic stem cell (HSC).

### (Item D15)

The composition any of the preceding items, wherein the migration and/or residual state is a migration from a niche of a bone marrow and/or a residual state at the niche of the bone marrow.

### (Item D16)

The composition of any of the preceding items, wherein the migration detecting agent comprises a detection agent for CD106 or a functional equivalent thereof.

### (Item D17)

A method for treating and/or preventing diabetes mellitus or a disease, disorder, and/or symptom associated with diabetes mellitus in a subject, the method comprising: administering an effective amount of an agent that reduces or eliminates an abnormal hematopoietic stem cell (HSC) and a stem cell migration agent to the subject.

### (Item D18)

A method for using a migration and/or residual state of an abnormal hematopoietic stem cell (HSC) as an indicator of treatment for treating and/or preventing diabetes mellitus or diabetes mellitus and/or a disease, disorder, and/or symptom associated with diabetes mellitus in a subject, the method comprising: detecting the migration and/or residual state of the abnormal hematopoietic stem cell (HSC) in the subject.

### (Item D19)

The composition of any of the preceding items, wherein the composition is a pharmaceutical composition.

### (Item D20)

The composition of any of the preceding items, further comprising a pharmaceutically acceptable excipient.

### (Item E1)

A method for treating and/or preventing diabetes mellitus and/or a disease, disorder, and/or symptom associated with diabetes mellitus in a subject, the method comprising: administering an effective amount of a suppressing agent for an abnormal hematopoietic stem cell (HSC) and a stem cell migration agent to the subject.

### (Item E2)

The method of any of the preceding items, wherein the abnormal HSC is a cell in which a gene or protein selected from the group consisting of CD106 and a functional equivalent thereof is not expressed and/or does not function at a normal level.

### (Item E3)

The method of any of the preceding items, wherein the expression which is not at a normal level is overexpression.

### (Item E4)

The method of any of the preceding items, wherein the suppressing agent comprises at least one selected from the group consisting of an anti-CD106 antibody or a functional variant thereof.

### (Item E5)

The method of any of the preceding items, wherein the abnormal HSC is a cell in which a gene or protein selected from the group consisting of tumor necrosis factor alpha (TNF-α), histone deacetylase (HDAC), and proinsulin is not expressed at a normal level.

### (Item E6)

The method of any of the preceding items, wherein the suppressing agent comprises at least one selected from the group consisting of an anti-TNF-α antibody or a functional variant thereof and an HDAC inhibiting agent (e.g., trichostatin A).

### (Item E7)

The method of any of the preceding items, wherein the disease, disorder, and/or symptom comprises a diabetic complication.

### (Item E8)

The method of any of the preceding items, wherein the disease, disorder, and/or symptom is selected from the group consisting of neuropathy, nephropathy, hepatopathy, retinopathy, fatty liver, gastrointestinal disorder, delayed bone fracture healing, eating disorder, and dermatopathy.

### (Item E9)

The method of any of the preceding items, wherein the stem cell migration agent has an ability to cause the abnormal HSC to migrate from a niche.

### (Item E10)

The method of any of the preceding items, wherein the stem cell migration agent comprises at least one agent selected from the group consisting of a CXCR4 antagonizing agent, a CXCR2 stimulating agent, an epidermal growth factor receptor (EGFR) inhibiting agent, and a granulocyte colony stimulating factor (G-CSF) agent.

### (Item E11)

The composition of any of the preceding items, wherein the stem cell migration agent comprises at least one selected from the group consisting of Plerixafor, GROβ2 (MIP2), Gefitinib, Erlotinib, Afatinib, Osimertinib, Filgrastim, Nartograstim, Lenograstim, and Pegfilgrastim.

### (Item E12)

A method for selecting a treatment for treating and/or preventing diabetes mellitus and/or a disease, disorder, and/or symptom associated with diabetes mellitus in a subject, the method comprising: administering an effective amount of an agent that detects a migration and/or residual state of an abnormal hematopoietic stem cell (HSC) to the subject.

### (Item E13)

The method of any of the preceding items, wherein the migration and/or residual state is a migration from a niche of a bone marrow and/or a residual state at the niche of the bone marrow.

### (Item E14)

The method of any of the preceding items, wherein the migration detecting agent comprises a detection agent for CD106 or a functional equivalent thereof.

### (Item E15)

A method for diagnosing a migration and/or residual state of an abnormal hematopoietic stem cell (HSC), or diabetes mellitus or diabetes mellitus and/or a disease, disorder, and/or symptom associated with diabetes mellitus in a subject, the method comprising: detecting the migration and/or residual state of the abnormal hematopoietic stem cell (HSC) in the subject.

### (Item F1)

A stem cell migration agent or an abnormal hematopoietic stem cell (HSC) suppressing agent for use in treating and/or preventing diabetes mellitus and/or a disease, disorder, and/or symptom associated with diabetes mellitus, for use in a combination of the stem cell migration agent and the abnormal HSC suppressing agent.

### (Item F2)

The stem cell migration agent or abnormal HSC suppressing agent of any of the preceding items, wherein the abnormal HSC is a cell in which a gene or protein selected from the group consisting of CD106 and a functional equivalent thereof is not expressed and/or does not function at a normal level.

### (Item F3)

The stem cell migration agent or abnormal HSC suppressing agent of any of the preceding items, wherein the expression which is not at a normal level is overexpression.

### (Item F4)

The suppressing agent of any of the preceding items, comprising at least one selected from the group consisting of an anti-CD106 antibody or a functional variant thereof.

### (Item F5)

The stem cell migration agent or abnormal HSC suppressing agent of any of the preceding items, wherein the abnormal HSC is a cell in which a gene or protein selected from the group consisting of tumor necrosis factor alpha (TNF-α), histone deacetylase (HDAC), and proinsulin is not expressed at a normal level.

### (Item F6)

The stem cell migration agent or abnormal HSC suppressing agent of any of the preceding items, comprising at least one selected from the group consisting of an anti-TNF-α antibody or a functional variant thereof and an HDAC inhibiting agent (e.g., trichostatin A).

### (Item F7)

The stem cell migration agent or abnormal HSC suppressing agent of any of the preceding items, wherein the disease, disorder, and/or symptom comprises a diabetic complication.

### (Item F8)

The stem cell migration agent or abnormal HSC suppressing agent of any of the preceding items, wherein the disease, disorder, and/or symptom is selected from the group consisting of neuropathy, nephropathy, hepatopathy, retinopathy, fatty liver, gastrointestinal disorder, delayed bone fracture healing, eating disorder, and dermatopathy.

### (Item F9)

The stem cell migration agent or abnormal HSC suppressing agent of any of the preceding items, wherein the stem cell migration agent has an ability to cause the abnormal HSC to migrate from a niche.

### (Item F10)

The stem cell migration agent or abnormal HSC suppressing agent of any of the preceding items, wherein the stem cell migration agent comprises at least one agent selected from the group consisting of a CXCR4 antagonizing agent, a CXCR2 stimulating agent, an epidermal growth factor receptor (EGFR) inhibiting agent, and a granulocyte colony stimulating factor (G-CSF) agent.

### (Item F11)

The stem cell migration agent or abnormal HSC suppressing agent of any of the preceding items, wherein the stem cell migration agent comprises at least one selected from the group consisting of Plerixafor, GROβ2 (MIP2), Gefitinib, Erlotinib, Afatinib, Osimertinib, Filgrastim, Nartograstim, Lenograstim, and Pegfilgrastim.

### (Item F13)

A stem cell migration agent for stem cell migration or an abnormal HSC inhibiting agent for use in treating and/or preventing diabetes mellitus and/or a disease, disorder, and/or symptom associated with diabetes mellitus, and for use in combination with a suppressing agent for an abnormal hematopoietic stem cell (HSC).

### (Item F14)

An agent for use in detection of a migration and/or residual state of an abnormal hematopoietic stem cell (HSC) for use in selection of a treatment for treating and/or preventing diabetes mellitus and/or a disease, disorder, and/or symptom associated with diabetes mellitus.

### (Item F15)

The agent of any of the preceding items, wherein the migration and/or residual state is a migration from a niche of a bone marrow and/or a residual state at the niche of the bone marrow.

### (Item F16)

The agent of any of the preceding items, comprising a detection agent for CD106 or a functional equivalent thereof.

### (Item G1)

Use of at least either one of an abnormal hematopoietic stem cell (HSC) suppressing agent and a stem cell migration agent in the manufacture of a medicament for treating and/or preventing diabetes mellitus and/or a disease, disorder, and/or symptom associated with diabetes mellitus, with a combination of the abnormal HSC suppressing agent and the stem cell migration agent.

### (Item G2)

The use of any of the preceding items, wherein the abnormal HSC is a cell in which a gene or protein selected from the group consisting of CD106 and a functional equivalent thereof is not expressed and/or does not function at a normal level.

### (Item G3)

The use of any of the preceding items, wherein the expression which is not at a normal level is overexpression.

### (Item G4)

The use of any of the preceding items, wherein the suppressing agent comprises at least one selected from the group consisting of an anti-CD106 antibody or a functional variant thereof.

### (Item G5)

The use of any of the preceding items, wherein the abnormal HSC is a cell in which a gene or protein selected from the group consisting of tumor necrosis factor alpha (TNF-α), histone deacetylase (HDAC), and proinsulin is not expressed at a normal level.

### (Item G6)

The use of any of the preceding items, wherein the suppressing agent comprises at least one selected from the group consisting of an anti-TNF-α antibody or a functional variant thereof and an HDAC inhibiting agent (e.g., trichostatin A).

### (Item G7)

The use of any of the preceding items, wherein the disease, disorder, and/or symptom comprises a diabetic complication.

### (Item G8)

The use of any of the preceding items, wherein the disease, disorder, and/or symptom is selected from the group consisting of neuropathy, nephropathy, hepatopathy, retinopathy, fatty liver, gastrointestinal disorder, delayed bone fracture healing, eating disorder, and dermatopathy.

### (Item G9)

The use of any of the preceding items, wherein the stem cell migration agent has an ability to cause the abnormal HSC to migrate from a niche.

### (Item G10)

The use of any of the preceding items, wherein the stem cell migration agent comprises at least one agent selected from the group consisting of a CXCR4 antagonizing agent, a CXCR2 stimulating agent, an epidermal growth factor receptor (EGFR) inhibiting agent, and a granulocyte colony stimulating factor (G-CSF) agent.

### (Item G11)

The use of any of the preceding items, wherein the stem cell migration agent comprises at least one selected from the group consisting of Plerixafor, GROβ2 (MIP2), Gefitinib, Erlotinib, Afatinib, Osimertinib, Filgrastim, Nartograstim, Lenograstim, and Pegfilgrastim.

### (Item G14)

Use of an agent for detecting a migration and/or residual state of an abnormal hematopoietic stem cell (HSC), in the manufacture of a medicament for use in selection of a treatment for treating and/or preventing diabetes mellitus and/or a disease, disorder, and/or symptom associated with diabetes mellitus.

### (Item G15)

The use of any of the preceding items, wherein the migration and/or residual state is a migration from a niche of a bone marrow and/or a residual state at the niche of the bone marrow.

### (Item G16)

The use of any of the preceding items, wherein the migration detecting agent comprises a detection agent for CD106 or a functional equivalent thereof.

The present disclosure is intended so that one or more of the aforementioned features can be provided not only as the explicitly disclosed combinations, but also as other combinations thereof. Additional embodiments and advantages of the present disclosure are recognized by those skilled in the art by reading and understanding the following detailed description as needed.

### [Advantageous Effects]

The present disclosure provides new therapy and diagnosis of diabetes mellitus and/or an associated disease thereof. The present disclosure can eliminate the necessity of donors conventionally required for pancreas transplantation, pancreatic islet transplantation, and the like, which are methods for recovering insulin secretion in patients with diabetes mellitus, and overcome the limitation in the supplying capacity compared to the number of recipients. The present disclosure can also avoid the burden on patients such as surgery that recipients have been subjected to. In this manner, the therapy of the present disclosure can be much easier and impose much lower burden because the therapy can be accomplished by administration of an agent. In addition, good prognosis is expected because radical therapy is possible.

### [Brief Description of Drawings]

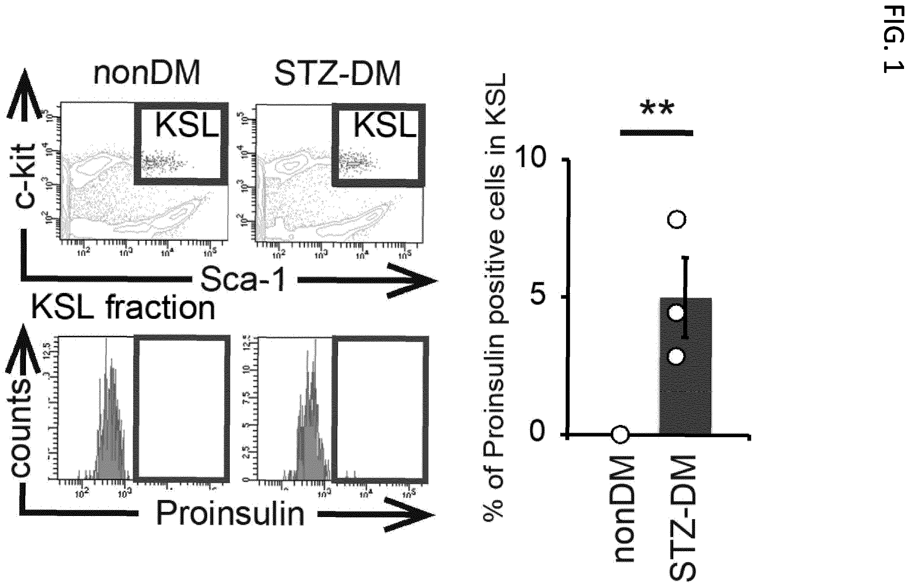

[Figure 1] Figure **1** shows fluorescence-activated cell sorting (FACS) analysis of proinsulin positive cells in a c-kit positive, Sca-1 positive, and lineage marker negative cell (KSL) fraction in mononuclear cells of non-diabetes mellitus (non-DM) and streptozotocin-induced diabetes mellitus (STZ-DM). The upper left two panels (left: non-DM, right: STZ-DM) show KSL cells (enclosed by a square) among lineage negative cells. The vertical axis indicates the fluorescence intensity of c-kit, while the horizontal axis indicates the fluorescence intensity of Sca-1. The lower left two panels (left: non-DM, right: STZ-DM) show the distribution of proinsulin positive cells (enclosed by a square). The vertical axis indicates the KSL cell count, while the horizontal axis indicates the fluorescence intensity of proinsulin. The graph on the right side (left: non-DM, right: STZ-DM) shows the ratio of proinsulin positive cells in KSL cells (n = 3 per group). **: P < 0.01.

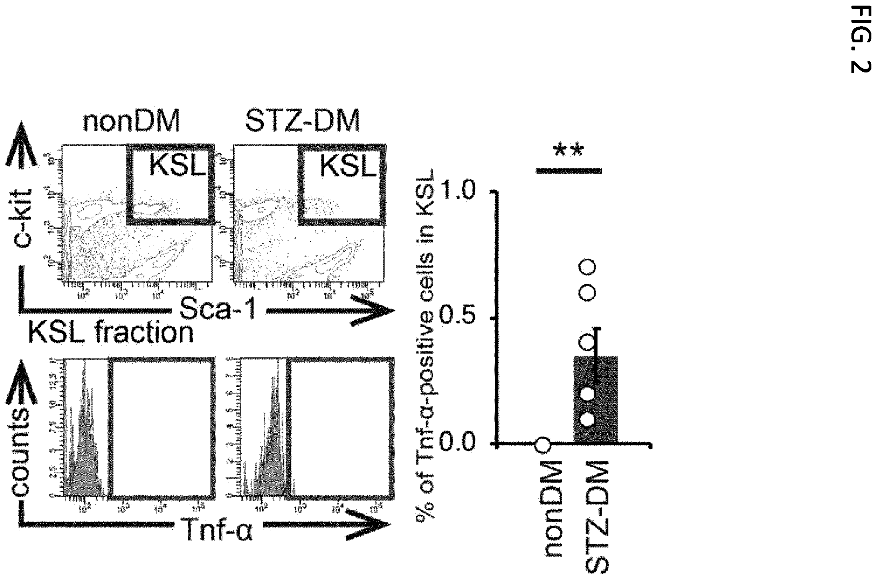

[Figure 2] Figure **2** shows FACS analysis of tumor necrosis factor alpha (TNF-α) positive cells in KSL cells in mononuclear cells of non-DM and STZ-DM. The upper left two panels (left: non-DM, right: STZ-DM) show KSL cells (enclosed by a square) among lineage negative cells. The vertical axis indicates the fluorescence intensity of c-kit, while the horizontal axis indicates the fluorescence intensity of Sca-1. The lower left two panels (left: non-DM, right: STZ-DM) show the distribution of TNF-α positive cells (enclosed by a square). The vertical axis indicates the KSL cell count, while the horizontal axis indicates the fluorescence intensity of TNF-α. The graph on the right side shows the ratio of TNF-α positive cells in KSL cells (n = 5 per group). The data is shown as average ± standard error. **: P < 0.01.

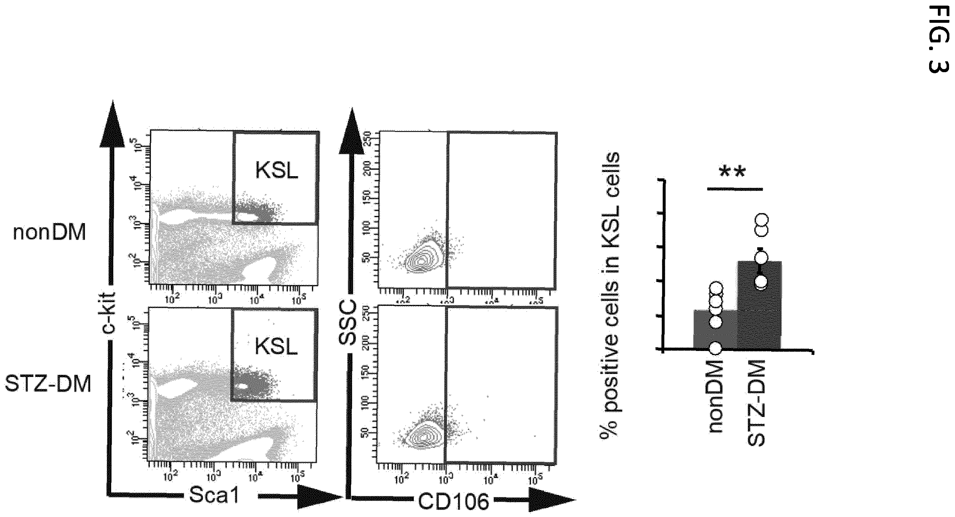

[Figure 3] Figure **3** shows FACS analysis of CD106 expression in KSL cells in mononuclear cells of non-DM and STZ-DM. The two panels on the left side (top: non-DM, bottom: STZ-DM) show KSL cells (enclosed by a square) among lineage negative cells. The vertical axis indicates the fluorescence intensity of c-kit, while the horizontal axis indicates the fluorescence intensity of Sca-1. The two panels on the right side (top: non-DM, bottom: STZ-DM) show the distribution of CD106 positive cells (enclosed by a square). The vertical axis indicates the intensity of side scatter light (SSC), while the horizontal axis indicates the fluorescence intensity of CD106. The graph on the right side (left: non-DM, right: STZ-DM) shows the ratio of CD106 positive cells in KSL cells (one division is equal to 1%) (n = 4 per group). **: P < 0.01.

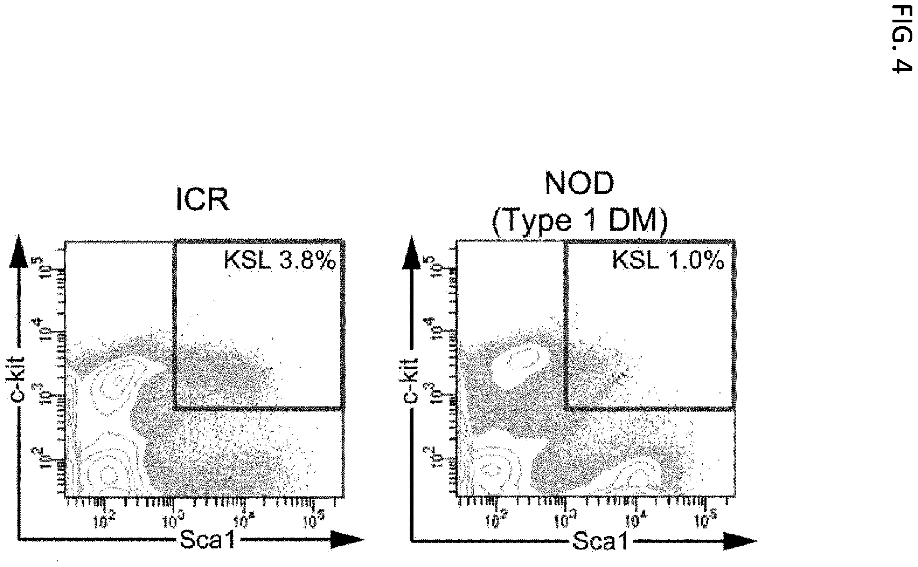

[Figure 4] Figure **4** shows FACS analysis of mononuclear cells of ICR mice and NOD mice. The two panels (left: ICR, right: NOD) show KSL cells (enclosed by a square) among lineage negative cells. The vertical axis indicates the fluorescence intensity of c-kit, while the horizontal axis indicates the fluorescence intensity of Sca-1.

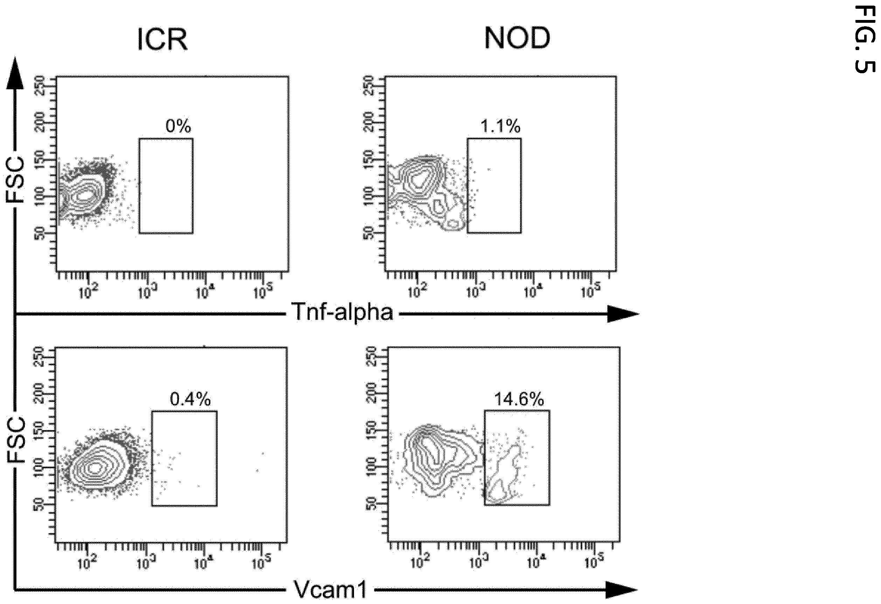

[Figure 5] Figure **5** shows FACS analysis of tumor necrosis factor alpha (TNF-α) positive cells and CD106 positive cells in KSL cells in mononuclear cells of ICR mice and NOD mice. The upper two panels (left: ICR, right: NOD) show the distribution of TNF-α positive cells (enclosed by a square). The vertical axis indicates the intensity of forward scatter light (FSC), while the horizontal axis indicates the fluorescence intensity of TNF-α. The lower two panels (left: ICR, right: NOD) show the distribution of CD106-posotive cells (enclosed by a square). The vertical axis indicates the intensity of forward scatter light (FSC), while the horizontal axis indicates the fluorescence intensity of CD106.

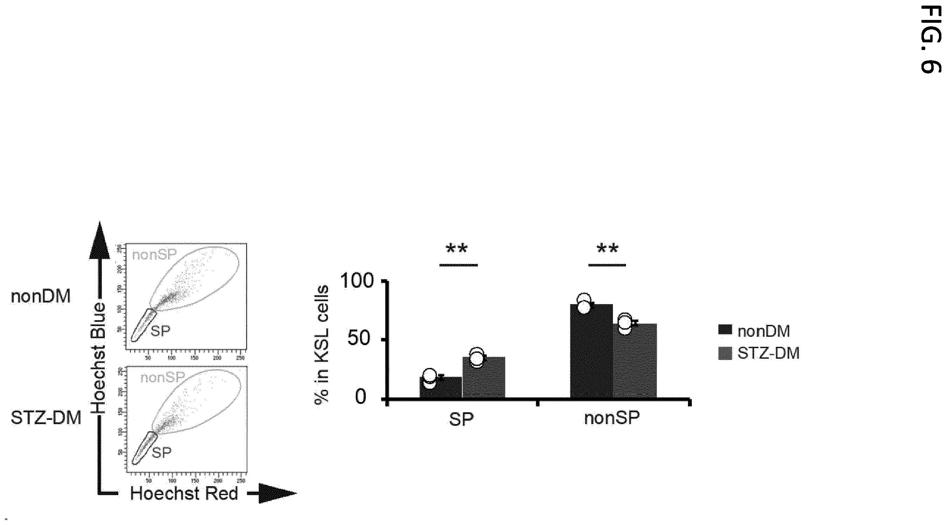

[Figure 6] Figure **6** shows FACS analysis of a side population (SP) fraction and a non-SP fraction in KSL cells of non-DM and STZ-DM. The two panels on the left side (top: non-DM, bottom: STZ-DM) show the SP fraction (enclosed by a lower left circle) and the non-SP fraction (enclosed by an upper right circle) in KSL cells. The vertical axis indicates the fluorescence intensity of Hoechst Blue, while the horizontal axis indicates the fluorescence intensity of Hoechst Red. The graph on the right side (left: SP, right: non-SP) shows the ratio of the SP fraction and the non-SP fraction in KSL cells of non-DM (left bar) and STZ-DM (right bar) (n = 3). The data is shown as average ± standard error. **: P < 0.01.

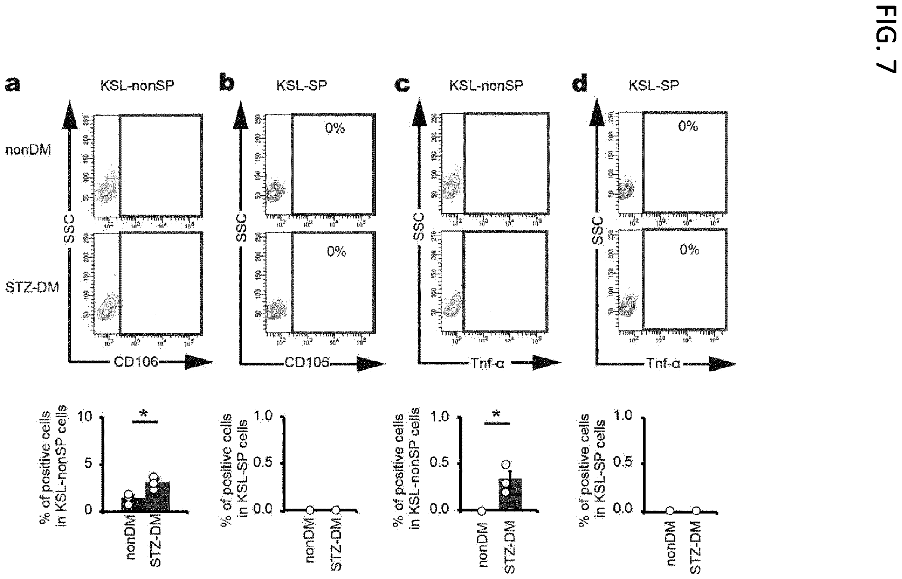

[Figure 7a] Figure **7a** shows FACS analysis of CD106 positive cells in KSL-non-SP cells of non-DM and STZ-DM. The two panels (top: non-DM, bottom: STZ-DM) show the distribution of CD106 positive cells (enclosed by a square). The vertical axis indicates the intensity of side scatter light (SSC), while the horizontal axis indicates the fluorescence intensity of CD106. The graph (left: non-DM, right: STZ-DM) shows the ratio of CD106 positive cells in KSL-non-SP cells (n = 3 per group).
[Figure 7b] Figure **7b** shows FACS analysis of CD106 positive cells in KSL-SP cells of non-DM and STZ-DM. The two panels (top: non-DM, bottom: STZ-DM) show the distribution of CD106 positive cells (enclosed by a square, which was 0%). The vertical axis indicates the intensity of side scatter light (SSC), while the horizontal axis indicates the fluorescence intensity of CD106. The graph (left: non-DM, right: STZ-DM) shows the ratio of CD106 positive cells in KSL-SP cells (n = 3 per group).
[Figure 7c] Figure **7c** shows FACS analysis of TNF-α positive cells in KSL-non-SP cells of non-DM and STZ-DM. The two panels (top: non-DM, bottom: STZ-DM) show the distribution of TNF-α positive cells (enclosed by a square). The vertical axis indicates the intensity of side scatter light (SSC), while the horizontal axis indicates the fluorescence intensity of TNF-α. The graph (left: non-DM, right: STZ-DM) shows the ratio of TNF-α positive cells in KSL-non-SP cells (n = 3 per group).
[Figure 7d] Figure **7d** shows FACS analysis of TNF-α positive cells in KSL-SP cells of non-DM and STZ-DM. The two panels (top: non-DM, bottom: STZ-DM) show the distribution of TNF-α positive cells (enclosed by a square, which was 0%). The vertical axis indicates the intensity of side scatter light (SSC), while the horizontal axis indicates the fluorescence intensity of TNF-α. The graph (left: non-DM, right: STZ-DM) shows the ratio of TNF-α positive cells in KSL-SP cells (n = 3 per group). The data is shown as average ± standard error. *: P < 0.05.

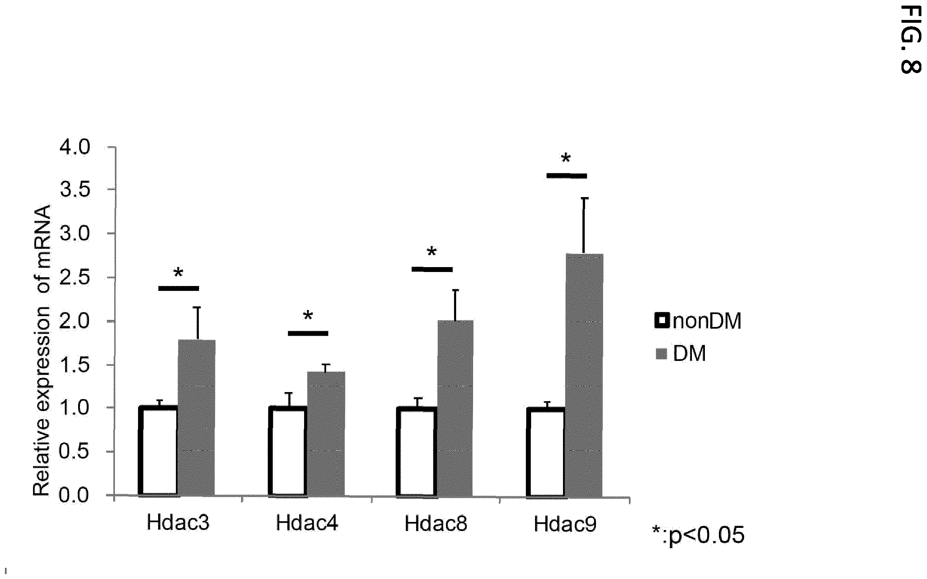

[Figure 8] Figure **8** shows comparison of the gene expression of histone deacetylase genes (HDACs) in KSL cells of non-DM mice and STZ-DM mice. Each bar graph shows, from the left, the amount of expression of Hdac3, Hdac4, Hdac8, and Hdac9. In each bar graph, the left bar shows the result of non-DM mice, while the right bar shows the result of STZ-DM mice. The vertical axis indicates the relative amount of the amount of mRNA expression in both types of mice when considering the amount of mRNA expression in the non-DM mice as 1. *: P < 0.05.

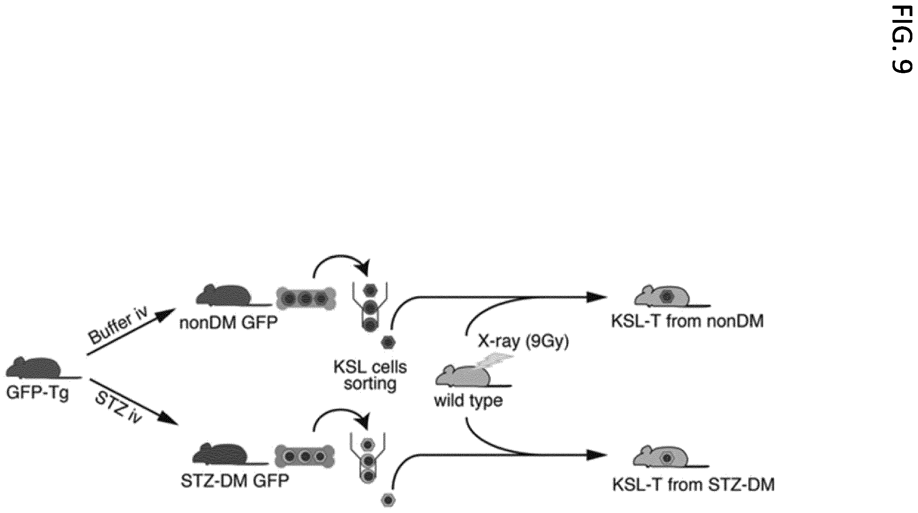

[Figure 9] Figure **9** shows the outline of the experiment for transplantation of non-DM mice- or STZ-DM mice-derived KSL cells to a normal blood glucose mouse. Transgenic mice into which green fluorescent protein had been transduced (GFP-Tg mice) were treated with STZ to prepare diabetes mellitus induced mice (STZ-DM GFP), and GFP-Tg mice were subjected to control treatment of an intravenous injection of a citrate buffer to prepare non-DM mice (non-DM GFP). After 3 months, KSL cells obtained from each of the non-DM mice and the STZ-DM mice were transplanted to normal blood glucose wild-type mice that had been irradiated with a lethal dose of radiation at 9 Gy (non-DM-derived KSL-T, STZ-DM-derived KSL-T) .

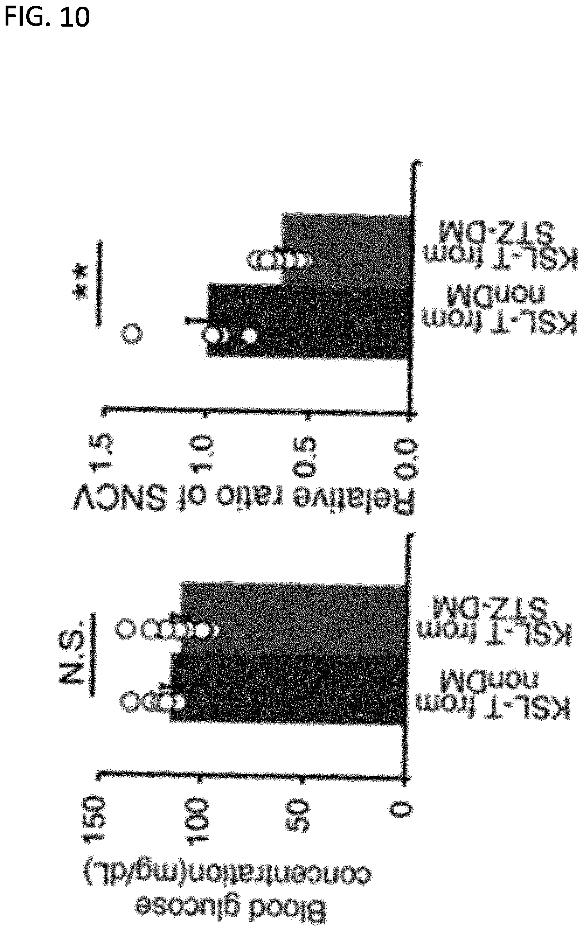

[Figure 10] Figure **10** shows the blood glucose concentration (mg/dL) (left) in non-DM-derived KSL-T (n = 9) and STZ-DM-derived KSL-T (n = 10) and the relative ratio (right) of the sensory nerve conduction velocity (SNCV) in the sciatic nerve when considering the measurement value in non-DM-derived KSL-T as 1. N.S indicates that there is no significant difference, and ** indicates P < 0.01.

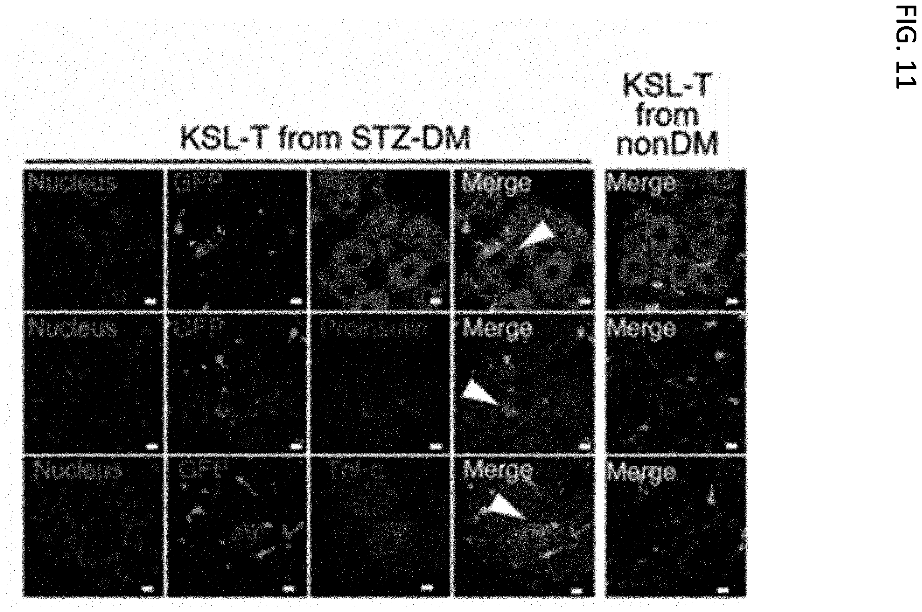

[Figure 11] Figure **11** shows immunofluorescence staining images of MAP 2, proinsulin, and TNF-α in a dorsal root ganglion (DRG). The four columns from the left show the results of STZ-DM-derived KSL-T, wherein each image shows, from the left, a nucleus (blue), GFP (green), target molecule (top row: MAP2, middle row: proinsulin, bottom row: TNF-α) (red), and merged image. The right column shows the results of non-DM-derived KSL-T, showing merged images of a nucleus (blue), GFP (green), and target molecule (top row: MAP2, middle row: proinsulin, bottom row: TNF-α) (red). The arrow heads indicate a fused cell. Scale bar = 10 µm.

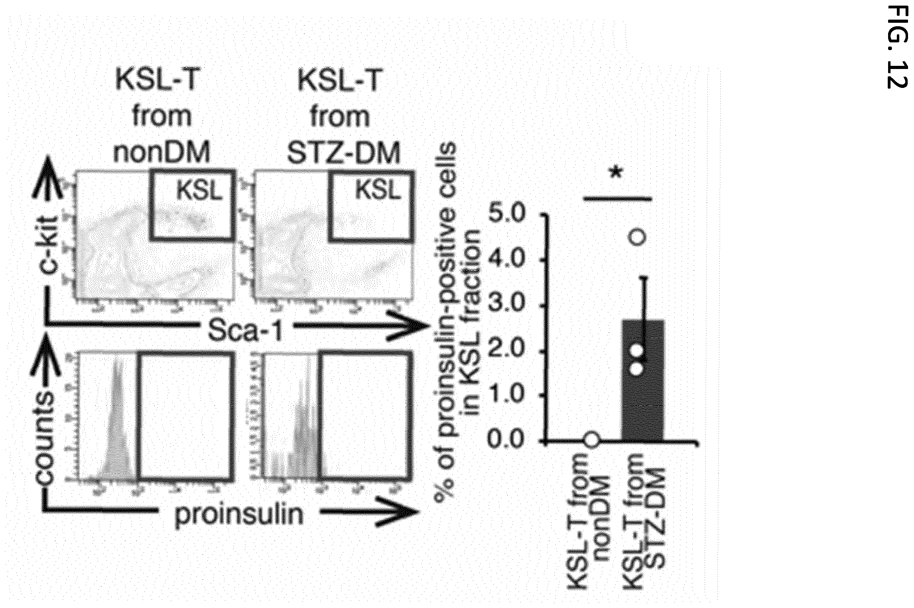

[Figure 12] Figure **12** shows FACS analysis of proinsulin positive cells in a KSL fraction in mononuclear cells of non-DM-derived KSL-T and STZ-DM-derived KSL-T. The upper left two panels (left: non-DM-derived KSL-T, right: STZ-DM-derived KSL-T) show KSL cells (enclosed by a square) among lineage negative cells. The vertical axis indicates the fluorescence intensity of c-kit, while the horizontal axis indicates the fluorescence intensity of Sca-1. The lower left two panels (left: non-DM-derived KSL-T, right: STZ-DM-derived KSL-T) show the distribution of proinsulin positive cells (enclosed by a square). The vertical axis indicates the KSL cell count, while the horizontal axis indicates the fluorescence intensity of proinsulin. The graph on the right side (left: non-DM-derived KSL-T, right: STZ-DM-derived KSL-T) shows the ratio of proinsulin positive cells in KSL cells (n = 3 per group). *: P < 0.05.

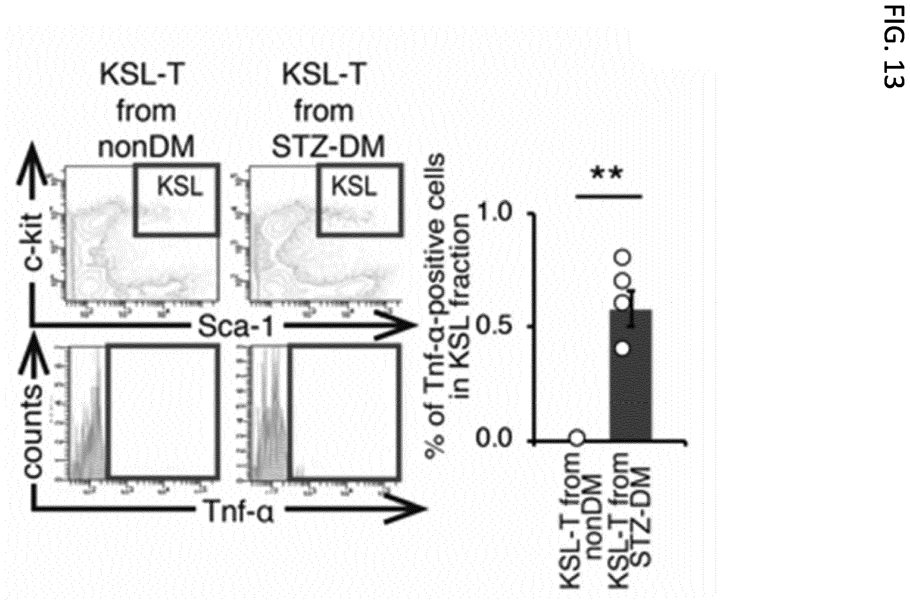

[Figure 13] Figure **13** shows FACS analysis of TNF-α positive cells in a KSL fraction in mononuclear cells of non-DM-derived KSL-T and STZ-DM-derived KSL-T. The upper left two panels (left: non-DM-derived KSL-T, right: STZ-DM-derived KSL-T) show KSL cells (enclosed by a square) among lineage negative cells. The vertical axis indicates the fluorescence intensity of c-kit, while the horizontal axis indicates the fluorescence intensity of Sca-1. The lower left two panels (left: non-DM-derived KSL-T, right: STZ-DM-derived KSL-T) show the distribution of TNF-α positive cells (enclosed by a square). The vertical axis indicates the KSL cell count, while the horizontal axis indicates the fluorescence intensity of TNF-α. The graph on the right side (left: non-DM-derived KSL-T, right: STZ-DM-derived KSL-T) shows the ratio of TNF-α positive cells in KSL cells (n = 3 per group). **: P < 0.01.

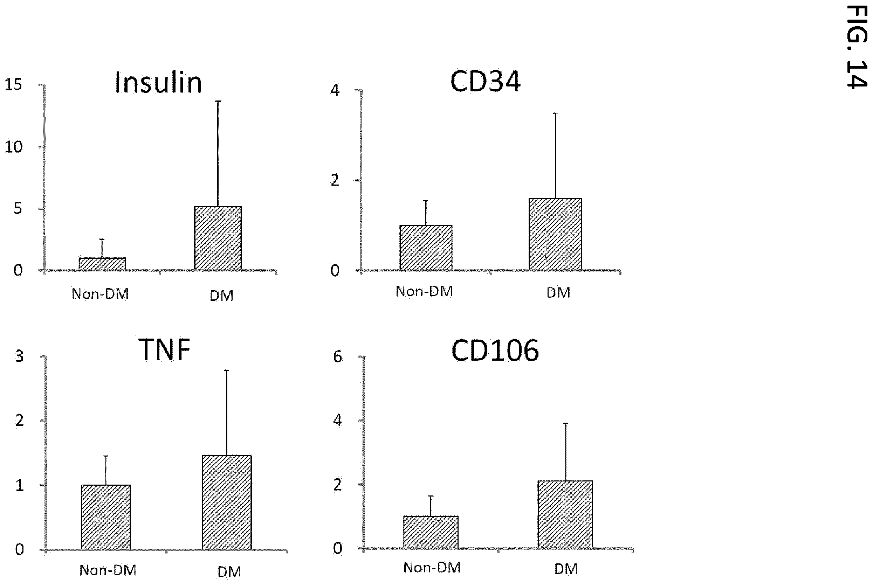

[Figure 14] Figure **14** shows comparison in the amount of expression of each gene in blood samples of human diabetic patients (DM) and healthy human subjects (non-DM). The panels each show insulin (upper left), CD34 (upper right), TNF-α (lower left), and CD106 (lower right). In each panel, the left bar shows the result of the healthy human subjects, while the right bar shows the result of the human diabetic patients. The vertical axis shows the relative amount of mRNA expression when considering the average result of the healthy human subjects as 1.

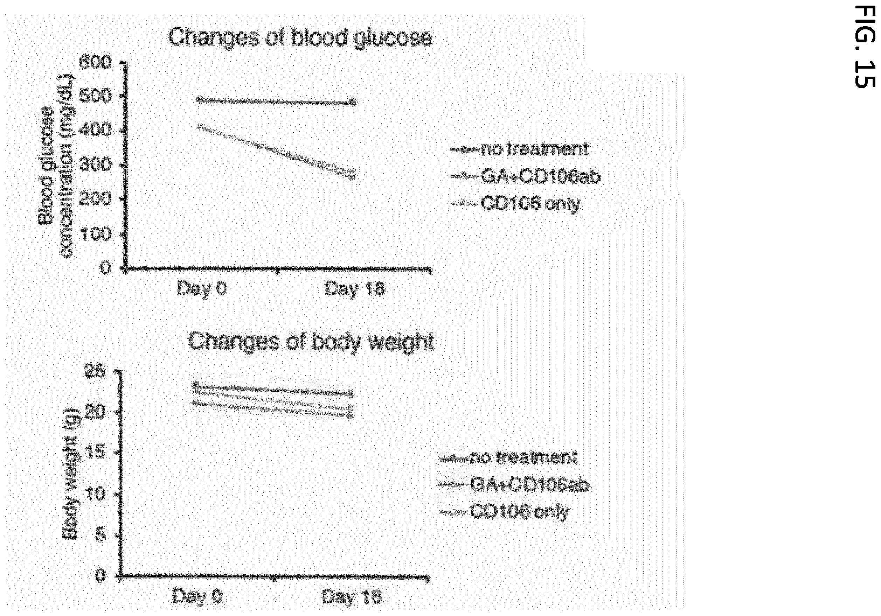

[Figure 15] Figure **15** shows the change in the blood glucose level and the body weight when streptozotocin-induced diabetes mellitus mice were treated with a stem cell migration agent and an anti-CD106 antibody (tail vein administration at a dose of 250 µg/mouse every week). The upper graph shows the blood glucose level (vertical axis, mg/dL) on day 0 and day 18. The lower graph shows the body weight (vertical axis, g) on day 0 and day 18. The blue line shows the result of control treatment. The orange line shows results of the stem cell migration agent and anti-CD106 antibody. The gray line shows the result of treatment with an anti-CD106 antibody.

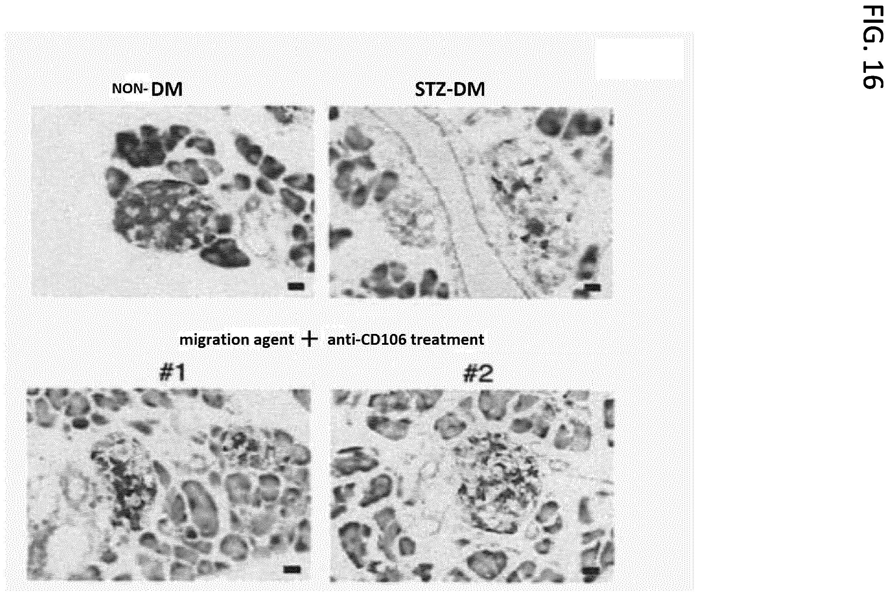

[Figure 16] Figure **16** shows stained images of insulin and nuclei of frozen mouse sections. The upper left shows a non-DM mouse, while the upper right shows an STZ-DM mouse. The bottom two show the results of two STZ-DM mice treated with a stem cell migration agent and an anti-CD106 antibody.

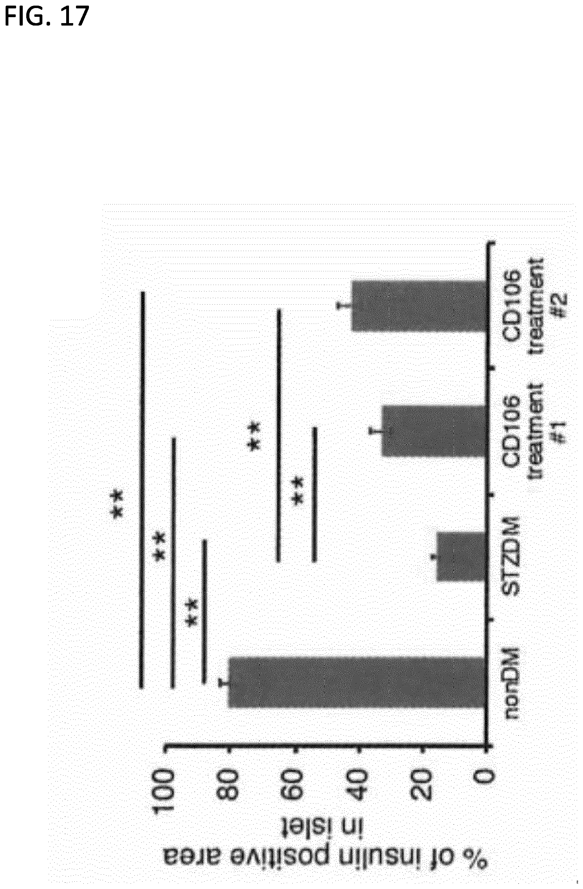

[Figure 17] Figure **17** shows the results obtained by randomly selecting about 8 to 10 islets in each mouse and calculating the ratio of the insulin positive area to the size of the islets by Image J software. From the left, shown are the results of a non-DM mouse, a STZ-DM mouse, and two STZ-DM mice (#1 and #2) treated with a stem cell migration agent and an anti-CD106 antibody. ** indicates P < 0.01.

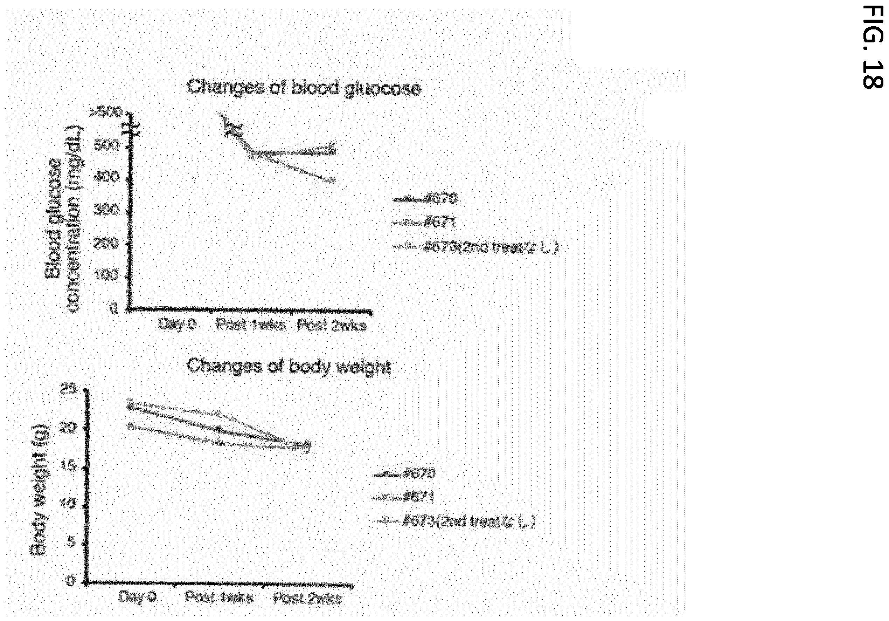

[Figure 18] Figure **18** shows the change in the blood glucose level and the body weight when NOD mice were treated with a stem cell migration agent and an anti-CD106 antibody (tail vein administration at a dose of 250 µg/mouse every week). The upper graph shows the blood glucose level (vertical axis, mg/dL) on day 7 and day 14. The lower graph shows the body weight (vertical axis, g) on day 7 and day 14. The blue line (# 670) and the orange line (# 671) show the results of two individual mice which received migration agent and anti-CD106 antibody treatment twice a week, respectively. The gray line (# 673) shows the results of individual mice which received the migration agent and anti-CD106 antibody treatment only once.

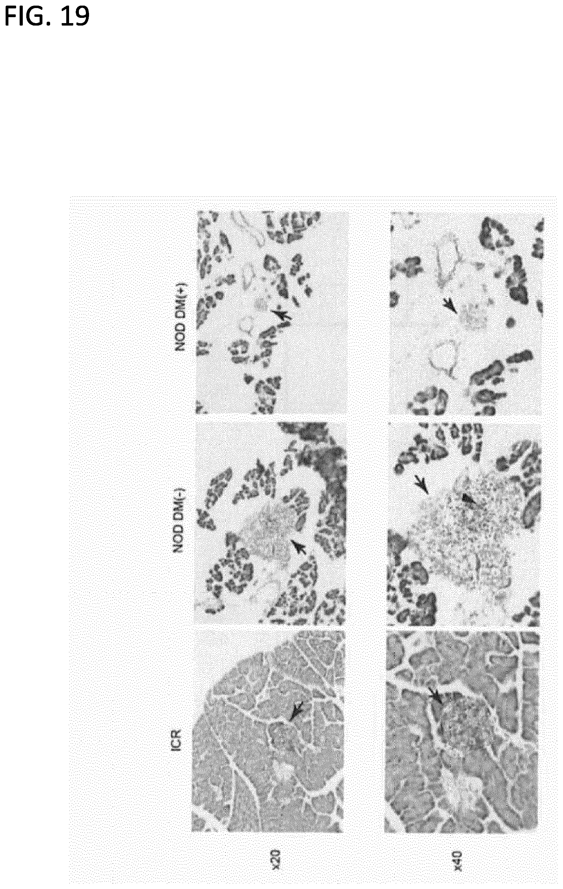

[Figure 19] Figure **19** shows stained images of insulin and nuclei of frozen mouse sections. From the left, shown are the results of a non-DM mouse, a NOD mouse before the onset of diabetes mellitus, and a NOD mouse after the onset of diabetes mellitus. The upper row is a 20x magnified image, while the lower row is a 40x magnified image. The arrowheads indicate pancreatic islets.

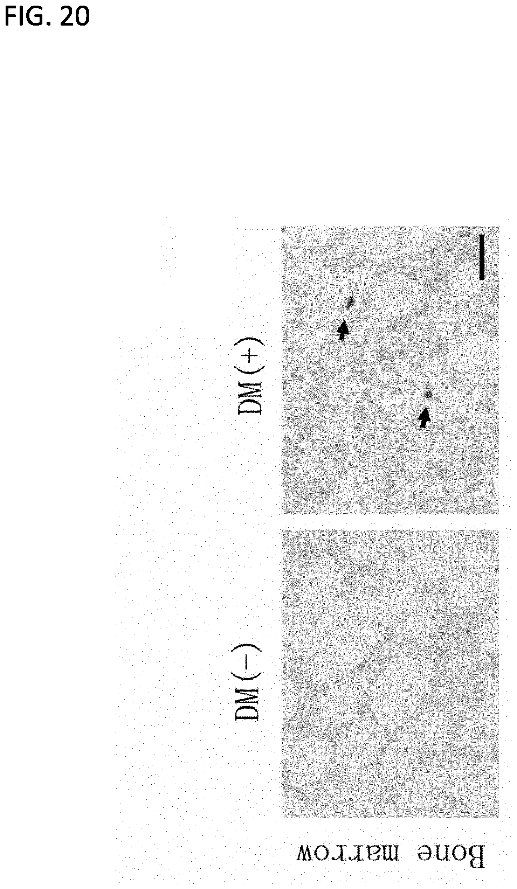

[Figure 20] Figure **20** shows proinsulin staining for the bone marrow of patients without DM (DM (-)) and patients with DM (DM (+)). Bone marrow cells stained with a proinsulin antibody (arrows) are observed in the case of DM (+), but not observed in the case of DM (-). The scale bar indicates 50 µm.

### [Description of Embodiments]

The present disclosure is described hereinafter while showing the best mode thereof. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence.

The definitions of the terms and/or basic technical concepts that are particularly used herein are described hereinafter when appropriate.

### (Definition)

As used herein, "diabetes mellitus" is used in the meaning that is commonly used in the art. Apart from diabetes mellitus due to pregnancy and diabetes mellitus due to specific gene abnormality, diabetes mellitus is generally classified into type 1 diabetes mellitus caused by depletion of insulin due to destruction of pancreatic β cells and type 2 diabetes mellitus caused by a decrease in the ability of glucose uptake into the muscle and fat tissue resulting from a reduction in the amount of insulin secreted from β cells of the islets of Langerhans (pancreatic islet) of the pancreas due to obesity or the like. The result of the present study suggests that both type 1 diabetes mellitus and type 2 diabetes mellitus are resultant from the same cellular cause via immune abnormality, which is completely different from the cause that was believed to be the cause of diabetes mellitus so far. Diabetes mellitus can be diagnosed by, for example, fasting blood glucose ≥ 126 mg/dL and HbA1c ≥ 6.5% in two or more tests, a 2-hour value of 200 mg/dL or greater in an oral glucose tolerance test (75 g OGTT), or the like. "Diabetes mellitus" as used herein also includes maturity onset diabetes of the young and prediabetes.

As used herein, an "associated disease of diabetes mellitus" or a "disease, disorder, and/or symptom associated with diabetes mellitus" can include any disease, disorder, and symptom associated with diabetes mellitus. Examples thereof include diabetic neuropathy, diabetic retinopathy, diabetic nephropathy, secondary diabetes mellitus, diabetic coma, disturbance of consciousness, stomachache, leg cramp, neuropathy, hyperosmolar hyperglycemic syndrome, albuminuria, edema, renal failure, blindness, Alzheimer's type dementia, myocardial infarction, obstructive arteriosclerosis, cerebral infarction, fatty liver, skin symptoms (such as diabetic lipoid necrobiosis), decrease in the wound healing ability, susceptibility to infections (such as septicemia), cancer (hepatic cancer, renal cancer, pancreatic cancer, colon cancer, gastric cancer, hepatic cancer, ovarian cancer, large intestine cancer, colon cancer, or the like), diabetic ketoacidosis, cardiac disease, cerebral vascular disorder, steroid diabetes mellitus, constipation, dizziness (orthostatic hypotension), erectile dysfunction, myocardial infarction, chest pain, severe appendicitis, peritoneal irritation symptom, low temperature burn, gestational diabetes mellitus, mouth dryness, polydipsia, polyuria, and the like.

As used herein, a "non-diabetic subject" means a subject who does not have diabetes mellitus or an associated disease thereof unless otherwise specified.

As used herein, "therapy" refers to the prevention of exacerbation, preferably maintaining of the current condition, more preferably alleviation, and still more preferably disappearance of a disease or disorder in case of such a condition, including being capable of exerting a prophylactic effect or an effect of improving a disease of a patient or one or more symptoms accompanying the disease. A suitable therapy based on preliminary diagnosis may be referred to as "companion therapy" and a diagnostic agent therefor may be referred to as "companion diagnostic agent".

As used herein, "prevention" refers to the action of taking a measure against a disease or disorder from being in such a condition prior to being in such a condition. For example, it is possible to use the agent of the present disclosure to perform diagnosis, and optionally use the agent of the present disclosure to prevent or take measures to prevent diabetes mellitus or the like.

As used herein, "diagnosis" refers to identifying various parameters associated with a condition (e.g., a disease or disorder) or the like in a subject to determine the current or future state of such a condition. The condition in the body can be investigated by using the method, composition, or system of the present disclosure. Such information can be used to select and determine various parameters of a formulation or method for the treatment or prevention to be administered, condition in a subject or the like. As used herein, "diagnosis" when narrowly defined refers to diagnosis of the current state, but when broadly defined includes "early diagnosis", "predictive diagnosis", "prediagnosis" and the like. Since the diagnostic method of the present disclosure in principle can utilize what comes out from a body and can be conducted away from a medical practitioner such as a physician, the present disclosure is industrially useful. In order to clarify that the method can be conducted away from a medical practitioner such as a physician, "assisting" "predictive diagnosis, prediagnosis or diagnosis" may be particularly recited with "assisted". The technology of the present disclosure can be applied to such diagnosis technology.

As used herein, "suppression" of a target cell means inducing a decrease in the growth rate of the target cell, damage to the target cell, a reduction in the target cell, and/or death of the target cell. Suppression of a target cell can be accomplished by a direct action and/or an indirect action. For example, the direct action of suppressing a target cell is accomplished by targeting any feature (e.g., expressed molecule) of the cell and inducing damage to the target cell, a reduction in the target cell, and/or death of the target cell. Examples thereof include, but are not limited to, those via mechanisms of irradiation, induction of apoptosis, inducing an attack by an immune cell, and the like. Examples of the indirect action of suppressing a target cell include, but are not limited to, a method of causing an immune system to learn recognizing and suppressing the target cell, and the like.

As used herein, a "suppressing agent" for a target cell means an agent that suppresses the target cell by any means. For a suppressing agent described herein, any means of suppressing a target cell can be used, and examples thereof include, but are not limited to, an attack by an immune cell which is caused by binding of a target cell to a targeting molecule (e.g., antibody), use of a radiation generating molecule, and the like.

As used herein, a "migration" of a cell means that the cell translocates into peripheral blood and/or circulating blood. Typically, migration means that cells translocate from the bone marrow (or specific sites therewithin) into peripheral blood and/or circulating blood. Such a specific site in the bone marrow may be referred to as a niche or a specific niche, and the specific niche refers to a microenvironment required for stem cells to maintain the properties thereof *in vivo.* Therefore, as used herein, the migration refers to, but is not limited to, migration of stem cells from a niche in certain embodiments.

As used herein, a "migration agent" for cells means an agent that causes a cell of interest to migrate by any means.

As used herein, a "hematopoietic stem cell" or "HSC" refers to a stem cell capable of differentiating into a blood cell. In a human adult, a hematopoietic stem cell is mainly present in the bone marrow and gives rise to a white blood cell (neutrophil, eosinophil, basophil, lymphocyte, monocyte, macrophage), a red blood cell, a platelet, a mast cell, and a dendritic cell. A human hematopoietic stem cell can be characterized by, for example, being CD34 positive and Thy-1 positive, and also can be characterized by being Lineage negative, CD34 positive, CD38 negative, CD90 positive, and CD45RA negative (i.e., Lin⁻CD34⁺CD38⁻CD90⁺CD45RA⁻) (e.g., see Proc Natl Acad Sci USA. 2011 Dec 13;108(50):20012-20017). A murine hematopoietic stem cell can be characterized as a c-kit positive, Sca-1 positive, and lineage marker negative (KSL) cell. A hematopoietic stem cell also can be characterized by being negative for both of two types of optical filters, Hoechst blue and Hoechst red when a bone marrow cell stained with Hoechest 33342 dye is excited with ultraviolet light (350 nm) and developed with these optical filters. A hematopoietic stem cell can be subclassified into a long-term hematopoietic stem cell (LT-HSC), a short-term hematopoietic stem cell (ST-HSC), and the like. For example, Cytometry Research 19(2) :25-32, 2009 can be referred to for the features of a hematopoietic stem cell.

As used herein, a "long-term hematopoietic stem cell (LT-HSC)" refers to a hematopoietic stem cell having a long-term bone marrow cell re-establishing ability. LT-HSC is generally identified with a surface antigen marker and can be expressed as CD34 negative, CD150 positive, CD48 negative, Lin negative, sca1 positive, and c-kit positive (or Lineage⁻c-Kit⁺Sca-1⁺CD34⁻/^{low}CD150⁺ cell) (e.g., in a mouse). LT-HSC also can be expressed as CD34 negative and CD38 negative (or CD34⁻CD38⁻ cell) (e.g., in a human) (e.g., Nat Immunol. 2010 Jul; 11(7) :585-93; Blood 108, 2446-2454, 2006) .

As used herein, a "short-term hematopoietic stem cell (ST-HSC)" refers to a hematopoietic stem cell having a short-term bone marrow re-establishing ability. ST-HSC is generally identified with a surface antigen marker and can be expressed as CD34 positive, CD150 positive, CD48 negative, Lin negative, sca-1 positive, and c-kit positive (e.g., in a mouse). ST-HSC also can be expressed as CD34 positive and CD38 negative (or CD34⁺CD38⁻ cell) (e.g., in a human) (e.g., Nat Immunol. 2010 Jul; 11(7) :585-93; Blood 108, 2446-2454, 2006) .

As used herein, an "abnormal hematopoietic stem cell" or "abnormal HSC" refers to a hematopoietic stem cell that expresses an abnormal function and/or lacks at least a part of a normal function. Typically, an abnormal HSC refers to a cell in which a gene or protein of CD106 or a functional equivalent thereof is not expressed and/or does not function at a normal level.

As used herein, a gene or protein being "not expressed at a normal level" refers to the gene or protein being expressed in an amount of or at a level that is not an amount or level of expression observed in a cell having a normal function. For example, regarding CD106, the case where CD106 deviates from the normal expression (normal value observed in a normal cell) can be determined to be abnormal. Preferably, the case where CD106 is expressed more than normal can be determined to be abnormal. For example, gene expression at a level that is not normal (e.g., overexpression) can be tested by sorting a KSL cell from a non-diabetic mouse and a diabetic mouse with FACS and extracting RNA, followed by comparing and analyzing the amount of gene expression by using QT-PCR. For example, protein expression at a level that is not normal can be tested by performing FACS analysis, detecting a surface antigen of an abnormal hematopoietic stem cell, and comparing a diabetic subject and a non-diabetic subject.

As used herein, a gene or protein "not functioning at a normal level" refers to a function at a level that is observed in a cell having a normal function not being observed in the gene or protein. For example, regarding CD106, the case where CD106 deviates from a level at which CD106 normally functions (normal level or value observed in a normal cell) can be determined to be abnormal. Preferably, the case where the function of CD106 is observed at a higher level than normal can be determined to be abnormal. For example, a protein not functioning at a normal level can be tested by performing FACS analysis, detecting a surface antigen of an abnormal hematopoietic stem cell, and comparing a diabetic subject and a non-diabetic subject to observe activation of the protein.

As used herein, "CXCR4" is a 7-transmembrane G protein-coupled receptor (GPCR), also known as CD184 or Fusin. The physiological ligand of CXCR4 is one of the CXC chemokines, which is stromal cell-derived factor-1 (SDF-1) that strongly induces the migration of monocytes and lymphocytes. Inhibition of CXCR4 can result in migration of hematopoietic stem cells (Future Oncol. 2007 Feb; 3(1): 19-27) .

As used herein, "CD106" is one type of surface antigen and is a type I membrane protein of a member of the Ig superfamily, which is also known as adhesion molecule VCAM-1 (Vascular cell adhesion molecule-1) or INCAM-100. CD106 is a cell surface sialoglycoprotein expressed by a cytokine-activated endothelium and is known to mediate white blood cell-endothelial cell adhesion and signaling. Representative examples in humans include VCAM-1 isoform a precursor (nucleic acid sequence: NM_001078.4, amino acid sequence: NP_001069.1), VCAM-1 isoform b precursor (nucleic acid sequence: NM_080682.2, amino acid sequence: NP_542413.1), and VCAM-1 isoform c precursor (nucleic acid sequence: NM_001199834.1, amino acid sequence: NP_001186763.1).

As used herein, "CD34" is a surface protein which is believed to be involved in attachment of a stem cell to a bone marrow extracellular matrix or a stromal cell. CD34 is known to be highly glycosylated, and phosphorylated by protein kinase C. Representative examples in humans include CD34 transcript variant 1 (nucleic acid sequence: NM_001025109.2, amino acid sequence: NP_001020280.1) and CD34 transcript variant 2 (nucleic acid sequence: NM_001773.3, amino acid sequence: NP_001764.1).

As used herein, "tumor necrosis factor alpha" or an abbreviation thereof, "TNF-α", refers to a protein that is mainly secreted by a macrophage and is a multifunctional proinflammatory cytokine belonging to the tumor necrosis factor (TNF) superfamily which is also known as TNF, DIF, TNFA, TNFSF2, and TNLG1F. TNF-α can function via receptors TNFRSF1A/TNFR1 and TNFRSF1B/TNFBR and is involved in regulating a wide range of biological processes such as cell growth, differentiation, apoptosis, lipid metabolism, and blood coagulation. Representative examples in humans include nucleic acid: NM_000594.4, amino acid sequence: NP_000585.2.

As used herein, "proinsulin" refers to a precursor protein of insulin. Proinsulin is processed in an endoplasmic reticulum of a pancreatic β cell, and the C-peptide region is removed in an immature secretory granule, whereby insulin consisting of an A chain and a B chain is produced. Representative examples in humans include proinsulin transcript variant 1 (nucleic acid sequence: NM_000207.3, amino acid sequence: NP_000198.1), proinsulin transcript variant 2 (nucleic acid sequence: NM_001185097.2, amino acid sequence: NP_001172026.1), proinsulin transcript variant 3 (nucleic acid sequence: NM_001185098.1, amino acid sequence: NP_001172027.1), and proinsulin transcript variant 4 (nucleic acid sequence: NM_001291897.2, amino acid sequence: NP_001278826.1).

As used herein, "c-Kit" refers to a type 3 transmembrane receptor for MGF (mast cell growth factor, also known as stem cell factor), which is also known as PBT, SCFR, KIT, CD117, or MASTC. Representative examples in humans include Kit isoform 1 precursor (nucleic acid sequence: NM_000222.2; amino acid sequence: NP_000213.1) and Kit isoform 2 precursor (nucleic acid sequence: NM_001093772.1; amino acid sequence: NP_001087241.1).

As used herein, "CD20" refers to a B lymphocyte surface molecule that is a member of the transmembrane 4A gene family, also known as MS4A1, B1, S7, Bp35, CD20, CVID5, MS4A2 or LEU-16, and plays a role in the development and differentiation of B cells into plasma cells. In humans, CD20 transcription variant 1 (nucleic acid sequence: NM_152866.2, amino acid sequence: NP_690605.1) and CD20 transcription variant 3 (nucleic acid sequence: NM_021950.3, amino acid sequence: NP_068769.2) are typical.

It is understood that referring to each protein herein is intended not only directed to a protein having (or a nucleic acid encoding) an amino acid sequence described in a particular accession number but also to a functional equivalent thereof, unless specifically noted otherwise.

It is understood that, as used herein, a "functional equivalent" of a certain molecule encompasses mutants or variants (e.g., amino acid sequence variant or the like) of the molecule which have a function as a feature (e.g., marker) described herein, those that exert the same function (not necessarily to the same degree) as the biological function of the molecule, and those that can change, upon action, into the molecule itself.

As used herein, a "functional variant" of a certain molecule encompasses substance resulting from altering the molecule while maintaining the function of the molecule (the degree of the function may be changed). Examples of a functional variant of a binding molecule such as an antibody include a substance conjugated with other portions (such as labels or other functional molecules (such as protein)) and a substance fragmented so as to maintain the function of binding with a target molecule. In this manner, as used herein, a functional variant maintains the function that is the basis prior to an alteration.

Those with amino acid sequences with one or more amino acid insertions, substitutions or deletions, or addition to one or both ends can be used as a functional equivalent in the present disclosure. As used herein, "one or more amino acid insertions, substitutions or deletions, or addition to one or both ends in an amino acid sequence" refers to an alteration with a substitution of a plurality of amino acids or the like to the extent that can occur naturally by a well-known technical method such as site-directed mutagenesis or by natural mutation.

As used herein, "biological function", when referring to a certain gene or a nucleic acid molecule or a polypeptide related thereto, refers to a specific function that the gene, the nucleic acid molecule or the polypeptide may have in a living body. Examples of such a function include, but are not limited to, production of a specific antibody, enzyme activity, impartation of resistance and the like. For such a biological activity, literatures and the like cited in the accession numbers, Entrez numbers, and the like mentioned in the table described above can be referred to, and such literatures and the like are also incorporated herein by reference. As used herein, biological function can be exerted by "biological activity". As used herein, "biological activity" refers to the activity possibly possessed by a certain agent (e.g., polynucleotide, protein or the like) in a living body. Biological activity encompasses an activity of exerting a variety of functions (e.g., transcription promoting activity), and also encompasses, for example, an activity of activating or inactivating another molecule by an interaction with a certain molecule. When two agents interact, biological activity thereof is a bond between the two molecules and a biological change induced thereby, for example, two molecules are considered to be bound together if precipitating one molecule using an antibody results in co-precipitation of the other molecule. Observation of such co-precipitation is one example of a determination approach. For example, when a certain agent is an enzyme, the biological activity thereof encompasses enzyme activity thereof. In another example, when a certain agent is a ligand, binding to a receptor corresponding to the ligand is encompassed. Such biological activity can be measured by a technique that is well known in the art.

A "derivative", "analog", or "mutant" of a protein or nucleic acid used herein includes, but is not intended to be limited to, molecules comprising a region substantially homologous to the protein or nucleic acid. Such a molecule, in various embodiments, is at least 30%, 40%, 50%, 60%, 70%, 80%, 90%, 95%, 98% or 99% identical throughout the amino acid sequence or nucleic acid sequence of the same size or in comparison to a sequence aligned by a homology computer program known in the art. Alternatively, a "derivative", "analog", or "mutant" of a nucleic acid can hybridize to the original nucleic acid under a stringent condition, moderately stringent condition, or non-stringent condition. Typically, a "derivative", "analog", or "mutant" of a protein refers to a product of subjecting a naturally-occurring protein to modification such as an amino acid substitution, deletion, and addition, a protein which exhibits the biological function of the naturally-occurring protein, although not necessarily to the same degree. For instance, the biological function of such a protein can be investigated by a suitable and available in vitro assay described herein or known in the art. While what is mainly discussed herein is regarding humans, it is understood that the discussion also applies to matters regarding other species (e.g., other species of primates) or animal species of other genera, and it is understood that matters regarding these mammals also remain within the scope of the present disclosure.

The term "activity" used herein refers to a function of a molecule in the broadest sense. Activity, although not intended to be limiting, generally includes a biological function, biochemical function, physical function, and chemical function of a molecule. Examples of activity include enzymatic activity, an ability to interact with another molecule, an ability to activate, promote, stabilize, inhibit, suppress, or destabilize a function of another molecule, stability, and an ability to localize at a specific position in a cell. When applicable, the term also relates to a function of a protein complex in the broadest sense.

A "functionally active" protein, polypeptide, fragment, or derivative as used herein has a structural function, regulatory function, or biochemical function of a protein such as biological activity.

As used herein, a "gene" refers to an agent defining a genetic trait. A gene is generally arranged in a certain order in a chromosome. A gene defining the primary structure of a protein is referred to as a structural gene, and a gene affecting the expression thereof is referred to as a regulator gene. As used herein, a "gene" may refer to a "polynucleotide", "oligonucleotide" and "nucleic acid" (including DNA, RNA, and the like herein). A "gene product" is a substance which is produced based on a gene, and refers to protein, mRNA, or the like. Thus, mRNA can also be included in the concept of a gene and can also fall under a gene product. "Expression of a gene" often refers to a transcription level such as mRNA.

As used herein, "protein", "polypeptide", "oligopeptide" and "peptide" are used herein in the same meaning and refer to an amino acid polymer of any length. The polymer may be straight, branched or cyclic. An amino acid may be a naturally-occurring, non-naturally occurring or altered amino acid. The term may also encompass those assembled into a complex of multiple polypeptide chains. The term also encompasses naturally-occurring or artificially altered amino acid polymers. Examples of such an alteration include disulfide bond formation, glycosylation, lipidation, acetylation, phosphorylation, and any other manipulation or alteration (e.g., conjugation with a labeling component). The definition also encompasses, for example, polypeptides comprising one or more analogs of an amino acid (e.g., including non-naturally occurring amino acids and the like), peptide-like compounds (e.g., peptoids) and other alterations known in the art.

As used herein, "polynucleotide", "oligonucleotide", and "nucleic acid" are used herein to have the same meaning and refer to a polymer of nucleotides of any length. The terms also encompass "oligonucleotide derivative" or "polynucleotide derivative". The "oligonucleotide derivative" and "polynucleotide derivative" are interchangeably used and refer to an oligonucleotide or polynucleotide comprising a derivative of a nucleotide or having a bond between nucleotides that is different from ordinary bonds. Specific examples of such oligonucleotides include: 2'-O-methyl-ribonucleotide; oligonucleotide derivatives with a phosphodiester bond in an oligonucleotide converted into phosphorothioate bond; oligonucleotide derivatives with a phosphodiester bond in an oligonucleotide converted into an N3'-P5' phosphoramidate bond; oligonucleotide derivatives with a ribose and a phosphodiester bond in an oligonucleotide converted into a peptide nucleic acid bond; oligonucleotide derivatives with a uracil in an oligonucleotide substituted with a C-5 propynyl uracil; oligonucleotide derivatives with a uracil in an oligonucleotide substituted with a C-5 thiazole uracil; oligonucleotide derivatives with a cytosine in an oligonucleotide substituted with a C-5 propynyl cytosine; oligonucleotide derivatives with a cytosine in an oligonucleotide substituted with a phenoxazine-modified cytosine; oligonucleotide derivatives with a ribose in DNA substituted with a 2'-O-propylribose; oligonucleotide derivatives with a ribose in an oligonucleotide substituted with a 2'-methoxyethoxy ribose; and the like. Unless noted otherwise, specific nucleic acid sequences are intended to encompass sequences that are explicitly set forth, as well as their conservatively altered variants (e.g., degenerate codon substitutes) and complementary sequences. Specifically, a degenerate codon substitute can be achieved by making a sequence in which the third position of one or more selected (or all) codons is substituted with a mixed base and/or deoxyinosine residue (Batzer et al., Nucleic Acid Res. 19: 5081 (1991); Ohtsuka et al., J. Biol. Chem. 260: 2605-2608 (1985); Rossolini et al., Mol. Cell. Probes 8: 91-98 (1994)). As used herein, "nucleic acid" is also interchangeably used with gene, cDNA, mRNA, oligonucleotide, and polynucleotide. As used herein, "nucleotide" may be naturally-occurring or non-naturally-occurring.

As used herein, "homology" of genes refers to the degree of identity of two or more genetic sequences with respect to one another, and having "homology" generally refers to having a high degree of identity or similarity. Therefore, the identity or similarity of sequences is higher when homology of two genes is high. Whether two types of genes have homology can be found by direct comparison of sequences or by a hybridization method under stringent conditions for nucleic acids. When two genetic sequences are directly compared, the genes are homologous typically if DNA sequences are at least 50% identical, preferably at least 70% identical, and more preferably at least 80%, 90%, 95%, 96%, 97%, 98%, or 99% identical between the genetic sequences. Thus, as used herein, "homolog" or "homologous gene product" refers to a protein in another species, preferably mammal, exerting the same biological function as a protein constituent of a complex which will be further described herein. Such a homolog may be also called "ortholog gene product".

Amino acids may be mentioned herein by either their commonly known three letter symbols or their one character symbols recommended by the IUPAC-IUB Biochemical Nomenclature Commission. Similarly, nucleotides may be mentioned by their commonly recognized one character codes. Comparison of similarity, identity, and homology of an amino acid sequence and a base sequence is calculated herein by using a sequence analysis tool BLAST with default parameters. For example, identity can be searched using BLAST 2.2.9 (published on May 12, 2004) of the NCBI. Herein, values for identity generally refer to a value obtained when aligned under the default conditions using BLAST described above. However, when a higher value is obtained by changing a parameter, the highest value is considered the value of identity. When identity is evaluated in a plurality of regions, the highest value thereamong is considered the value of identity. Similarity is a value calculated by taking into consideration a similar amino acid in addition to identity.

As used herein, "polynucleotide which hybridizes under a stringent condition" refers to commonly used, well-known conditions in the art. Such a polynucleotide can be obtained by using colony hybridization, plaque hybridization, Southern blot hybridization, or the like while using a polynucleotide selected from the polynucleotides of the present disclosure as a probe. Specifically, the polynucleotide refers to a polynucleotide that can be identified by using a filter with immobilized DNA from a colony or plaque and performing hybridization at 65°C in the presence of 0.7 to 1.0 M NaCl, and then using an SSC (saline-sodium citrate) solution with 0.1 to 2× concentration (composition of an SSC solution with 1× concentration is 150 mM sodium chloride and 15 mM sodium citrate) to wash the filter under the condition of 65°C. Hybridization can be performed in accordance with the method described in experimental publications such as Molecular Cloning 2nd ed., Current Protocols in Molecular Biology, Supplement 1-38, DNA Cloning 1: Core Techniques, A Pracltical Approach, Second Edition, Oxford University Press (1995). A lowly stringent condition includes performing hybridization for 18 to 20 hours at 40°C in a buffer comprising 35% formamide, 5 × SSC, 50 mM Tris-HCl (pH of 7.5), 5 mM EDTA, 0.02% polyvinylpyrrolidone (PVP), 0.02% BSA, 100 µg/ml denatured salmon sperm DNA, and 10% (weight/volume) dextran sulfate, washing for 1 to 5 hours at 55°C in a buffer comprising 2 × SSC, 25 mM Tris-HCl (pH of 7.4), 5 mM EDTA, and 0.1% SDS, and washing for 1.5 hours at 60°C in a buffer comprising 2 × SSC, 25 mM Tris-HCl (pH of 7.4), 5 mM EDTA, and 0.1% SDS.

As used herein, a "purified" substance or biological agent (e.g., nucleic acid, protein or the like) refers to a substance or a biological agent wherein at least a part of an agent naturally accompanying the biological agent has been removed. Thus, the purity of a biological agent in a purified biological agent is generally higher than the purity in the normal state of the biological agent (i.e., concentrated). The term "purified" as used herein refers to the presence of preferably at least 75% by weight, more preferably at least 85% by weight, still more preferably at least 95% by weight, and most preferably at least 98% by weight of a biological agent of the same type. The substance used in the present disclosure is preferably a "purified" substance.

As used herein, a "corresponding" amino acid or nucleic acid refers to an amino acid or a nucleotide which has or is expected to have, in a certain polypeptide molecule or polynucleotide molecule, similar action as a predetermined amino acid or nucleotide in a reference polypeptide or a polynucleotide for comparison, and, particularly in the case of enzyme molecules, refers to an amino acid which is present at a similar position in an active site and makes a similar contribution to catalytic activity. For example, for an antisense molecule, it can be a similar moiety in an ortholog corresponding to a specific moiety of the antisense molecule. A corresponding amino acid can be a specific amino acid subjected to, for example, cysteination, glutathionylation, S-S bond formation, oxidation (e.g., oxidation of methionine side chain), formylation, acetylation, phosphorylation, glycosylation, myristylation or the like. Alternatively, a corresponding amino acid can be an amino acid responsible for dimerization. Such a "corresponding" amino acid or nucleic acid may be a region or a domain over a certain range. Thus, it is referred herein as a "corresponding" region or domain in such a case.

As used herein, a "corresponding" gene (e.g., polynucleotide sequence or molecule) refers to a gene (e.g., polynucleotide sequence or molecule) in a certain species which has or is expected to have similar action as a predetermined gene in a reference species for comparison. When there is a plurality of genes having such action, the corresponding gene refers to a gene having the same evolutionary origin. Hence, a gene corresponding to a certain gene may be an ortholog of such a gene. Such a corresponding gene can be identified by using a technique that is well known in the art. For example, a corresponding gene in a certain animal (e.g., mouse or rat) can be found by search on a sequence database for the animal using the sequence of a reference gene of the corresponding gene (e.g., gene of humans) as a query sequence.

As used herein, a "fragment" refers to a polypeptide or polynucleotide with a sequence length of 1 to n-1 with respect to the full length polypeptide or polynucleotide (with length n). The length of a fragment can be appropriately changed in accordance with the objective. Examples of the lower limit of such a length include 3, 4, 5, 6, 7, 8, 9, 10, 15, 20, 25, 30, 40, 50 and more amino acids for a polypeptide. Lengths represented by an integer that is not specifically listed herein (e.g., 11 and the like) also can be suitable as a lower limit. Further, examples of the length include 5, 6, 7, 8, 9, 10, 15, 20, 25, 30, 40, 50, 75, 100, and more nucleotides for a polynucleotide. Lengths represented by an integer that is not specifically listed herein (e.g., 11 and the like) also can be suitable as a lower limit. As used herein, such a fragment is understood to be within the scope of the present disclosure, for example, when a full length version functions as a marker, as long as the fragment itself also functions as a marker. As used herein, a fragment of a molecule is a substance (typically a polypeptide) comprising any region of the molecule, which does not need to have the biological function of a naturally-occurring molecule as long as the fragment can be used for the objective of the present disclosure (e.g., therapy, detection, diagnosis, or the like).

As used herein, "expression" of a gene, a polynucleotide, a polypeptide, or the like refers to the gene or the like being subjected to a certain action in vivo to be converted into another form. Preferably, expression refers to a gene, a polynucleotide, or the like being transcribed and translated into a form of a polypeptide. However, transcription to make an mRNA also can be one embodiment of expression. More preferably, such a polypeptide form can be a form which has undergone post-translation processing (derivative mentioned herein). For example, the expression level of a molecule can be determined by any method. Specifically, the expression level can be studied by evaluating the amount of mRNA, the amount of protein, or the biological activity of protein of the molecule.

As used herein, "amount of expression" refers to the amount of polypeptide, mRNA or the like expressed in a cell, tissue or the like of interest. Examples of such an amount of expression include the amount of expression of the polypeptide of the present disclosure at a protein level assessed by any suitable method including an immunological measurement method such as ELISA, RIA, fluorescent antibody method, western blot, and immunohistochemical staining by using the antibody of the present disclosure, and the amount of expression of the polypeptide used in the present disclosure at an mRNA level assessed by any suitable method including a molecular biological measuring method such as northern blot, dot blot, and PCR. "Change in an amount of expression" refers to an increase or decrease in the amount of expression of the polypeptide used in the present disclosure at a protein level or mRNA level assessed by any suitable method including the above-described immunological measuring method or molecular biological measuring method. A variety of detection or diagnosis based on a marker can be performed by measuring the amount of expression of a certain marker.

As used herein, a "marker (substance, protein or gene (nucleic acid))" refers to a substance that can be an indicator for tracking whether a subject is in or at risk of being in a certain condition (e.g., type of cell, normal cell state, diseased state, disorder state, or level of ability to grow, differentiation state, or the like). Examples of such a marker can include genes (nucleic acid = DNA level), gene products (such as mRNA or protein), metabolites, enzymes and the like. In the present disclosure, detection, diagnosis, preliminary detection, prediction, or prediagnosis of a certain state (such as diabetes mellitus) can be materialized by using an agent or means specific to a marker associated with such a state, or a composition, kit or system comprising the same or the like.

As used herein, an "antibody" includes, in a broad sense, polyclonal antibodies, monoclonal antibodies, multi-specific antibodies, chimeric antibodies, anti-idiotype antibodies, and fragments thereof such as Fv fragments, Fab' fragments, F(ab')₂ and Fab fragments, as well as other conjugates or functional equivalents produced by recombination (e.g., chimeric antibodies, humanized antibodies, multifunctional antibodies, bispecific or oligospecific antibodies, single chain antibodies, scFV, diabodies, sc(Fv)₂ (single chain (Fv)₂), and scFv-Fc). Furthermore, such an antibody may be fused, by a covalent bond or recombination, with an enzyme such as alkaline phosphatase, horseradish peroxidase, or α galactosidase. The origin, type, shape or the like of the antibody used in the present disclosure does not matter. Specifically, known antibodies such as a non-human animal antibody (e.g., a mouse antibody, a rat antibody, or a camel antibody), a human antibody, a chimeric antibody, or a humanized antibody can be used. In the present disclosure, a monoclonal or polyclonal antibody can be utilized as an antibody, but a monoclonal antibody is preferable. It is preferable that an antibody binds specifically to a target protein.

As used herein, "means" refers to anything that can be a tool for accomplishing an objective (e.g., detection, diagnosis, therapy, prevention, or migration). As used herein, "selective recognizing (detecting) means" in particular refers to means capable of recognizing (detecting) a certain subject differently from others.

As used herein, "detection" or "quantification" of polynucleotide or polypeptide expression can be accomplished by using a suitable method including, for example, an immunological measuring method and measurement of mRNAs, including a bond or interaction to a marker detection agent. Examples of a molecular biological measuring method include northern blot, dot blot, PCR and the like. Examples of an immunological measurement method include ELISA using a microtiter plate, RIA, fluorescent antibody method, luminescence immunoassay (LIA), immunoprecipitation (IP), single radial immunodiffusion (SRID), turbidimetric immunoassay (TIA), western blot, immunohistochemical staining and the like. Further, examples of a quantification method include ELISA, RIA and the like. Quantification may also be performed by a gene analysis method using an array (e.g., DNA array, protein array). DNA arrays are outlined extensively in (Ed. by Shujunsha, Saibo Kogaku Bessatsu "DNA Maikuroarei to Saishin PCR ho" [Cellular engineering, Extra issue, "DNA Microarrays and Latest PCR Methods*"*]. Protein arrays are discussed in detail in Nat Genet. 2002 Dec; 32 Suppl: 526-32. Examples of a method of analyzing gene expression include, but are not limited to, RT-PCR, RACE, SSCP, immunoprecipitation, two-hybrid system, in vitro translation and the like, in addition to the methods discussed above. Such additional analysis methods are described in, for example, Genomu Kaiseki Jikkenho Nakamura Yusuke Labo Manyuaru [Genome analysis experimental method Yusuke Nakamura Lab Manual], Ed. by Yusuke Nakamura, Yodosha (2002) and the like. The entirety of the descriptions therein is incorporated herein by reference.

The detection agent or detecting means of the present disclosure may be a complex or complex molecule in which another substance (e.g., label) is bound to a detectable part (e.g., antibody or the like). As used herein, a "complex" or "complex molecule" refers to any structure comprising two or more parts. For example, when one part is a polypeptide, the other part may be a polypeptide or other substance (e.g., substrate, sugar, lipid, nucleic acid, other carbohydrate or the like). Two or more constituent parts of the complex herein may be covalently bonded or bonded by other bonds (e.g., hydrogen bond, ionic bond, hydrophobic interaction, van der Waals force or the like). When two or more parts are polypeptides, a complex may be called a chimeric polypeptide. Thus, the "complex" herein encompasses molecules made by linking multiple types of molecules such as polypeptides, polynucleotides, lipids, sugars, or small molecules.

The detection agent or other medicaments of the present disclosure can be in the form of a probe or a primer. The probe or primer of the present disclosure can specifically hybridize with a target nucleic acid molecule. The probe and primer of the present disclosure only need to be able to detect the expression of a target nucleic acid molecule and can be a polymer consisting of a plurality of bases or base pairs such as deoxyribonucleic acid (DNA) or ribonucleic acid (RNA). It is known that a double stranded cDNA also can be utilized in tissue in situ hybridization. The probe and primer of the present disclosure also include such a double stranded cDNA. Examples of particularly preferred probe and primer in detection of RNA in tissue can include an RNA probe (riboprobe).

The primer and primer set according to the present disclosure can be utilized in a known method for detecting a gene of interest by utilizing a nucleic acid amplifying method such as PCR, RT-PCR, real-time PCR, in situ PCR, or LAMP as a primer and a primer set in accordance with a common method.

As used herein, a "probe" refers to a substance that can be means for search, which is used in a biological experiment such as in vitro and/or in vivo screening. Examples thereof include, but are not limited to, a nucleic acid molecule comprising a specific base sequence, a peptide comprising a specific amino acid sequence, a specific antibody, a fragment thereof and the like. As used herein, a probe is used as means for marker detection.

A nucleic acid molecule generally used as a probe includes those having a nucleic acid sequence with a length of at least 8 contiguous nucleotides, which is homologous or complementary to a nucleic acid sequence of a gene of interest. Such a nucleic acid sequence may be a nucleic acid sequence with a length of preferably at least 9 contiguous nucleotides, more preferably at least 10 contiguous nucleotides, still more preferably at least 11 contiguous nucleotides, at least 12 contiguous nucleotides, at least 13 contiguous nucleotides, at least 14 contiguous nucleotides, at least 15 contiguous nucleotides, at least 20 contiguous nucleotides, at least 25 contiguous nucleotides, at least 30 contiguous nucleotides, at least 40 contiguous nucleotides, or at least 50 contiguous nucleotides. A nucleic acid sequence used as a probe comprises a nucleic acid sequence that is at least about 70% homologous, more preferably at least about 80% homologous, still more preferably at least about 90% homologous, or at least about 95% homologous with the aforementioned sequence.

In one embodiment, the detection agent of the present disclosure can be labeled. Alternatively, the detection agent of the present disclosure may be a detection agent to which a tag is bound.

As used herein, a "label" refers to an entity (e.g., substance, energy, electromagnetic wave or the like) for distinguishing a molecule or substance of interest from others. Examples of such a method of labeling can include RI (radioisotope) method, fluorescence method, biotin method, chemiluminescent method and the like. When a plurality of markers of the present disclosure or agents or means for capturing the same are labeled by a fluorescence method, labeling is performed with fluorescent substances having different fluorescent emission maximum wavelengths. It is preferable that the difference in fluorescent emission maximum wavelengths is 10 nm or greater. When labeling a ligand, any label that does not affect the function can be used, but Alexa^{™} Fluor is desirable as a fluorescent substance. Alexa^{™} Fluor is a water-soluble fluorescent dye obtained by modifying coumarin, rhodamine, fluorescein, cyanine or the like. This is a series compatible with a wide range of fluorescence wavelengths. Relative to other fluorescent dyes for the corresponding wavelength, Alexa^{™} Fluor is very stable, bright and has a low level of pH sensitivity. Combinations of fluorescent dyes with fluorescence maximum wavelength of 10 nm or greater can include a combination of Alexa^{™} 555 and Alexa^{™} 633, combination of Alexa^{™} 488 and Alexa^{™} 555 and the like. When a nucleic acid is labeled, any substance that can bind to a base portion thereof can be used. However, it is preferable to use a cyanine dye (e.g., Cy3, Cy5 or the like of the CyDye^{™} series), rhodamine 6G reagent, N-acetoxy-N2-acetylaminofluorene (AAF), AAIF (iodine derivative of AAF) or the like. Examples of a fluorescent substance with a difference in fluorescent emission maximum wavelengths of 10 nm or greater can include a combination of Cy5 and a rhodamine 6G reagent, a combination of Cy3 and fluorescein, a combination of a rhodamine 6G reagent and fluorescein and the like. The present disclosure can utilize such a label to alter a subject of interest to be detectable by the detecting means to be used. Such alteration is known in the art. Those skilled in the art can appropriately carry out such a method in accordance with the label and subject of interest.

As used herein, "tag" refers to a substance for distinguishing a molecule by a specific recognition mechanism such as receptor-ligand, or more specifically, a substance serving the role of a binding partner for binding a specific substance (e.g., having a relationship such as biotin-avidin or biotin-streptavidin). A tag can be encompassed in the scope of "label". Accordingly, for example, a specific substance to which a tag is bound can distinguish the specific substance by contact with a substrate, to which a binding partner of a tag sequence is bound. Such a tag or label is well known in the art. Typical tag sequences include, but are not limited to, myc tag, His tag, HA, Avi tag and the like. Such a tag may be bound to the marker or marker detection agent of the present disclosure.

As used herein, an "agent" is used broadly and may be any substance or other elements (e.g., light, radiation, heat, electricity and other forms of energy) as long as the intended objective can be achieved. Examples of such a substance include, but are not limited to, cell (e.g., T cell), protein, polypeptide, oligopeptide, peptide, polynucleotide, oligonucleotide, nucleotide, nucleic acid (including for example DNAs such as cDNA and genomic DNA, RNAs such as mRNA), polysaccharide, oligosaccharide, lipid, organic small molecule (e.g., hormone, ligand, information transmitting substance, organic small molecule, molecule synthesized by combinatorial chemistry, small molecule that can be used as medicine (e.g., small molecule ligand and the like), and the like) and a composite molecule thereof.

As used herein, a "kit" refers to a unit generally providing portions to be provided (e.g., inspection drug, diagnostic drug, therapeutic drug, antibody, label, manual and the like) into two or more separate sections. This form of a kit is preferred when a composition that should not be provided in a mixed state and is preferably mixed immediately before use for safety or the like is intended to be provided. Preferably, such a kit advantageously comprises an instruction or manual describing how the provided portions (e.g., inspection drug, diagnostic drug, or therapeutic drug) are used or how a reagent should be handled. When the kit is used herein as a reagent kit, the kit generally comprises an instruction or the like describing how to use an inspection drug, diagnostic drug, therapeutic drug, antibody and the like.

As used herein, an "instruction" is a document with an explanation of the method of use of the present disclosure for a physician or other users. The instruction has a description of the detection method of the present disclosure, method of use of a diagnostic agent, or administration of a medicament or the like. Further, an instruction may have a description instructing oral administration or administration to the esophagus (e.g., by injection or the like) as a site of administration. The instruction is prepared in accordance with a format defined by the regulatory agency of the country in which the present disclosure is practiced (e.g., the Ministry of Health, Labor and Welfare in Japan, Food and Drug Administration (FDA) in the U.S. or the like), with an explicit description showing approval by the regulatory agency. The instruction is a so-called package insert and is typically provided in, but not limited to, paper media. The instruction may also be provided in a form such as electronic media (e.g., web sites provided on the Internet or emails).

As used herein, an "amount" of an analyte or the like in a sample generally refers to an absolute value reflecting the mass of the analyte that can be detected in a volume of the sample. However, an amount is also intended as a relative amount as compared to another analyte amount. For example, the amount of an analyte in a sample can be an amount that is greater than a control value or a normal value of the analyte that is generally present in the sample.

As used herein, a "level" of an analyte or the like in a sample generally refers to an absolute value reflecting the value of activity or the like of the analyte when the analyte is a subject exerting a function such as enzymes. However, a level is also intended as a relative level as compared to another analyte level. For example, the level of an analyte in a sample can be a level that is greater than a control value or a normal value of the analyte that is generally present in the sample.

As used herein, the term "about" refers to the indicated value plus or minus 10%. Even when "about" is not explicitly mentioned for a value, the value can be interpreted synonymously with a value with "about".

### (Summary of the present disclosure)

The present inventors have found a new aspect in which the effect of diabetes mellitus therapy targeting abnormal stem cells characterized by abnormal expression of CD106 and the like, can be improved in combination with stem cell migration. The present disclosure provides new therapeutic strategies and diagnoses for diabetes mellitus and/or associated disease thereof based on the new diabetes mellitus therapeutic strategy.

### (Preferred embodiments)

Preferred embodiments of the present disclosure are described below. Embodiments provided below are provided to facilitate the understanding of the present disclosure. It is understood that the scope of the present disclosure should not be limited to the following descriptions. Thus, it is apparent that those skilled in the art can make appropriate modifications within the scope of the present disclosure by referring to the descriptions herein. It is understood that the following embodiments of the present disclosure can be used alone or in combination.

### (Features of an abnormal stem cell related to diabetes mellitus and/or associated disease thereof)

In one embodiment, the abnormal stem cell of interest in the present disclosure can be a hematopoietic stem cell. In one embodiment, the abnormal stem cell of the present disclosure is characterized in that at least one of CD106, CD34, TNF-α, proinsulin, and histone deacetylases (HDACs) is not expressed and/or does not function at a normal level, and optionally, the abnormal stem cell of the present disclosure can be further characterized by having other features of the abnormal stem cell described herein (e.g., being c-Kit positive, being Sca-1 positive, being hematopoietic stem cell lineage marker negative, features of a short-term hematopoietic stem cell described above (such as being CD38 negative)). In one embodiment, the abnormal stem cell of the present disclosure can be characterized by not expressing CD106 at a normal level. In one embodiment, the abnormal stem cell of the present disclosure is characterized by (a) not expressing CD106 at a normal level and (b) not expressing at a normal level at least one of CD34 abnormal expression, TNF-α abnormal expression, proinsulin abnormal expression, and histone deacetylases (HDACs) abnormal expression, and optionally, the abnormal stem cell of the present disclosure can be further characterized by having other features of the abnormal stem cell described herein (e.g., being c-Kit positive, being Sca-1 positive, being hematopoietic stem cell lineage marker negative, features of a short-term hematopoietic stem cell described above (such as being CD38 negative)).

In one representative embodiment, the abnormal stem cell (e.g., hematopoietic stem cell) of interest in the present disclosure can be characterized by abnormal expression of CD106. In one embodiment, the abnormal stem cell of the present disclosure can be characterized by a higher amount of CD106 expression than the amount of CD106 expression in whole bone marrow cells or hematopoietic stem cells (e.g., CD34 positive and Thy-1 positive cells) in a population of non-diabetic subjects. In one embodiment, the abnormal stem cell of the present disclosure can be characterized by expressing 1 × 10² or greater, 2 × 10² or greater, 5 × 10² or greater, 1 × 10³ or greater, 2 × 10³ or greater, 5 × 10³ or greater, 1 × 10⁴ or greater, 2 × 10⁴ or greater, 5 × 10⁴ or greater, 1 × 10⁵ or greater, 2 × 10⁵ or greater, 5 × 10⁵ or greater, 1 × 10⁶ or greater, 2 × 10⁶ or greater, 5 × 10⁶ or greater, or 1 × 10⁷ or greater CD106 molecules per cell on the cell surface. In a specific embodiment, the abnormal stem cell of the present disclosure can be characterized by expressing about 1 × 10⁴ or greater CD106 molecules per cell on the cell surface. In one embodiment, the abnormal stem cell of the present disclosure can be indicated by the amount of expression that exhibits a fluorescence intensity which is 1-fold or greater, 2-fold or greater, 5-fold or greater, 10-fold or greater, 20-fold or greater, 50-fold or greater, 100-fold or greater, 200-fold or greater, 500-fold or greater, or 1000-fold or greater the median of the fluorescence intensity derived from the fluorescent dye observed in whole bone marrow cells or hematopoietic stem cells when whole bone marrow cells or hematopoietic stem cells (e.g., CD34 positive and Thy-1 positive cells) of a normal subject (e.g., human) are labeled with a fluorescent dye-conjugated antibody for CD106 and analyzed by FACS. In one embodiment, the abnormal stem cell of the present disclosure can be characterized by the amount of expression of mRNA of CD106 which is higher than that of hematopoietic stem cells (e.g., CD34 positive cells) derived from a population of non-diabetic subjects by 10% or greater, 20% or greater, 30% or greater, 40% or greater, 50% or greater, 60% or greater, 70% or greater, 80% or greater, 90% or greater, 100% or greater, 150% or greater, or 200% or greater.

In one embodiment, the abnormal stem cell of interest in the present disclosure can be characterized by expression of TNF-α. In one embodiment, the abnormal stem cell of the present disclosure can be characterized by being positive for expression of TNF-α. In one embodiment, being positive for expression of TNF-α can be indicated by the amount of expression that exhibits a fluorescence intensity which is 1-fold or greater, 2-fold or greater, 5-fold or greater, 10-fold or greater, 20-fold or greater, 50-fold or greater, 100-fold or greater, 200-fold or greater, 500-fold or greater, or 1000-fold or greater the median of the fluorescence intensity derived from the fluorescent dye observed in whole bone marrow cells or hematopoietic stem cells when whole bone marrow cells or hematopoietic stem cells (e.g., CD34 positive and Thy-1 positive cells) of a normal subject (e.g., human) are labeled with a fluorescent dye-conjugated antibody for TNF-α and analyzed by FACS. In one embodiment, the abnormal stem cell of the present disclosure can be characterized by the amount of expression of mRNA of TNF-α which is higher than that of hematopoietic stem cells (e.g., CD34 positive cells) derived from a population of non-diabetic subjects by 10% or greater, 20% or greater, 30% or greater, 40% or greater, 50% or greater, 60% or greater, 70% or greater, 80% or greater, 90% or greater, 100% or greater, 150% or greater, or 200% or greater.

In one embodiment, the abnormal stem cell of interest in the present disclosure can be characterized by abnormal expression of proinsulin. In one embodiment, the abnormal stem cell of the present disclosure can be characterized by being positive for expression of proinsulin. In one embodiment, being positive for expression of proinsulin can be indicated by the amount of expression that exhibits a fluorescence intensity which is 1-fold or greater, 2-fold or greater, 5-fold or greater, 10-fold or greater, 20-fold or greater, 50-fold or greater, 100-fold or greater, 200-fold or greater, 500-fold or greater, or 1000-fold or greater the median of the fluorescence intensity derived from the fluorescent dye observed in whole bone marrow cells or hematopoietic stem cells when whole bone marrow cells or hematopoietic stem cells (e.g., CD34 positive and Thy-1 positive cells) of a non-diabetic subject (e.g., human) are labeled with a fluorescent dye-conjugated antibody for proinsulin and analyzed by FACS. In one embodiment, the abnormal stem cell of the present disclosure can be characterized by the amount of expression of mRNA of insulin (or proinsulin) which is higher than that of hematopoietic stem cells (e.g., CD34 positive cells) derived from a population of non-diabetic subjects by 10% or greater, 20% or greater, 30% or greater, 40% or greater, 50% or greater, 60% or greater, 70% or greater, 80% or greater, 90% or greater, 100% or greater, 150% or greater, 200% or greater, 300% or greater, 400% or greater, 500% or greater, 700% or greater, or 1000% or greater.

In one embodiment, the abnormal stem cell of interest in the present disclosure can be characterized by expression of c-Kit. In one embodiment, the abnormal stem cell of the present disclosure can be characterized by being positive for expression of c-Kit. In one embodiment, being positive for expression of c-Kit can be indicated by the amount of expression that exhibits a fluorescence intensity which is 0.1-fold or greater, 0.2-fold or greater, 0.5-fold or greater, 1-fold or greater, 2-fold or greater, 5-fold or greater, 10-fold or greater, 20-fold or greater, 50-fold or greater, 100-fold or greater, 200-fold or greater, 500-fold or greater, or 1000-fold or greater the median of the fluorescence intensity derived from the fluorescent dye observed in whole bone marrow cells or hematopoietic stem cells when whole bone marrow cells or hematopoietic stem cells (e.g., CD34 positive and Thy-1 positive cells) of a non-diabetic subject (e.g., human) are labeled with a fluorescent dye-conjugated antibody for c-Kit and analyzed by FACS, and/or exhibits a fluorescence intensity which remains within the top 70%, 60%, 50%, 40%, 30%, 20%, or 10% when the cells are ranked in descending order in the fluorescence intensity derived from the fluorescent dye thereof.

In one embodiment, the abnormal stem cell of interest in the present disclosure can be characterized by expression of Sca-1. In one embodiment, the abnormal stem cell of the present disclosure can be characterized by being positive for expression of Sca-1. In one embodiment, being positive for expression of Sca-1 can be indicated by the amount of expression that exhibits a fluorescence intensity which is 0.1-fold or greater, 0.2-fold or greater, 0.5-fold or greater, 1-fold or greater, 2-fold or greater, 5-fold or greater, 10-fold or greater, 20-fold or greater, 50-fold or greater, 100-fold or greater, 200-fold or greater, 500-fold or greater, or 1000-fold or greater the median of the fluorescence intensity derived from the fluorescent dye observed in whole bone marrow cells when whole bone marrow cells of a non-diabetic subject (e.g., a human) are labeled with a fluorescent dye-conjugated antibody for Sca-1 and analyzed by FACS, and/or exhibits a fluorescence intensity which remains within top 70%, 60%, 50%, 40%, 30%, 20%, or 10% when the whole bone marrow cells are ranked in descending order in the fluorescence intensity derived from the fluorescent dye thereof.

In one embodiment, the abnormal stem cell of interest in the present disclosure can be characterized by expression of a hematopoietic stem cell lineage marker. Examples of a hematopoietic stem cell lineage marker include CD3 (T cell), CD19 (B cell), NK1.1 (NK cell), CD11c (dendritic cell), CD11b (monocyte), FcεRI (mast cell), Gr-1 (granulocyte), and the like. In one embodiment, the abnormal stem cell of the present disclosure can be characterized by being negative for expression of one or more (e.g., all) of CD3, CD19, NK1.1, CD11c, CD11b, FcεRI, and Gr-1. In one embodiment, being negative for expression of each of CD3, CD19, NK1.1, CD11c, CD11b, FcεRI, and Gr-1 can be indicated by the amount of expression that exhibits a fluorescence intensity which is 1000% or less, 500% or less, 200% or less, 100% or less, 50% or less, 20% or less, 10% or less, 5% or less, 2% or less, or 1% or less of the median of the fluorescence intensity derived from the fluorescent dye observed in whole bone marrow cells when whole bone marrow cells of a non-diabetic subject (e.g., human) are labeled with a fluorescent dye-conjugated antibody for CD3, CD19, NK1.1, CD11c, CD11b, FcεRI, or Gr-1 and analyzed by FACS, and/or exhibits a fluorescence intensity which remains within the bottom 50%, 20%, 10%, 5%, 2%, or 1% when the whole bone marrow cells are ranked in descending order in the fluorescence intensity derived from the fluorescent dye thereof. In one embodiment, the abnormal stem cell of the present disclosure can be characterized by the amount of expression of mRNA of CD34 which is higher than that of hematopoietic stem cells derived from a population of non-diabetic subjects by 10% or greater, 20% or greater, 30% or greater, 40% or greater, 50% or greater, 60% or greater, 70% or greater, 80% or greater, 90% or greater, 100% or greater, 150% or greater, or 200% or greater.

In one embodiment, the abnormal stem cell of interest in the present disclosure can be characterized by being a hematopoietic stem cell. In one embodiment, being a human hematopoietic stem cell can also be characterized by being CD34 positive and Thy-1 positive or being Lineage negative, CD34 positive, CD38 negative, CD90 positive, and CD45RA negative. In one embodiment, a murine hematopoietic stem cell can be characterized by being c-kit positive, Sca-1 positive, and lineage marker negative (KSL). In one embodiment, being a hematopoietic stem cell can also be characterized by being negative for both of two types of optical filters, Hoechst blue and Hoechst red when a bone marrow cell stained with Hoechest 33342 dye is excited with ultraviolet light (350 nm) and developed with these filtered.

In one embodiment, the abnormal stem cell of interest in the present disclosure can be characterized by a cell stage of a hematopoietic stem cell. In one embodiment, the abnormal stem cell of the present disclosure can be a short-term hematopoietic stem cell.

In one embodiment, the abnormal stem cell of interest in the present disclosure can be characterized by expression of histone deacetylase genes (HDACs). In one embodiment, the abnormal stem cell of the present disclosure can be characterized by the amount of expression of one or more of histone deacetylase genes (HDACs) (e.g., Hdac3, Hdac4, Hdac8, and Hdac9) which is higher than that of hematopoietic stem cells (e.g., c-Kit positive lineage marker negative cells) derived from a population of non-diabetic subjects by 10% or greater, 20% or greater, 30% or greater, 40% or greater, 50% or greater, 60% or greater, 70% or greater, 80% or greater, 90% or greater, 100% or greater, 150% or greater, 200% or greater, 300% or greater, 400% or greater, or 500% or greater.

In one embodiment, proinsulin and/or TNF-α can be a marker indicating the localization of the abnormal stem cells of interest in the present disclosure in the bone marrow.

### (Therapy and/or prevention of diabetes mellitus and/or a disease, disorder, and/or symptom associated with diabetes mellitus, in combination with stem cell migration, targeting an abnormal stem cell)

In one aspect, the present disclosure provides therapy and/or prevention of diabetes mellitus and/or a disease, disorder, and/or symptom associated with diabetes mellitus by causing to migrate and suppressing an abnormal stem cell having a feature described herein. The therapy and/or prevention can be accomplished by any means such as performing a method or providing a composition, combination, kit, or system for the objective. For example, when an suppressing agent such as an antibody is used, the suppressing effect on cells present in the bone marrow may be limiting (see, for example, the Examples). Therefore, as in the present disclosure, by causing abnormal stem cells to migrate before suppressing the abnormal stem cells, the effect of suppressing the abnormal stem cells can be improved.

In one embodiment, the therapy and/or prevention of the present disclosure can be performed by causing to migrate and suppressing all cells having at least one of the features of the abnormal stem cell described herein. In other words, a cell that is caused to migrate and is suppressed in the present embodiment is not limited to the abnormal stem cell described herein. It can be technically difficult to cause to migrate and suppress only the abnormal stem cell described herein, and therapy and/or prevention can be selected while taking into consideration the benefit derived from the migration and suppression of an abnormal stem cell and the effect of the migration and suppression of other cells. Meanwhile, the migration and suppression of some of the abnormal stem cells described herein may be insufficient for therapy and/or prevention of diabetes mellitus and/or a disease, disorder, and/or symptom associated with diabetes mellitus. Thus, it can be preferable to cause to migrate and suppress all of the abnormal stem cells described herein. In the therapy and/or prevention of the present disclosure, the features of the abnormal stem cells utilized for migration and the features of the abnormal stem cells utilized for suppression may be the same or different. For example, in one embodiment, the therapy and/or prevention of the present disclosure may utilize, for migration, the feature that abnormal stem cells are hematopoietic stem cells (for example, using a CXCR4 antagonizing agent and/or a CXCR2 stimulating agent), and may utilize, for suppression, the feature that abnormal stem cells abnormally express certain cell surface proteins (such as CD106) (for example, using an anti-CD106 antibody).

In one embodiment, the migration in the therapy and/or prevention of the present disclosure can be performed by causing abnormal stem cells having any of the features described herein to migrate. In one embodiment, the migration of abnormal stem cells described herein can be performed by treatment for causing hematopoietic stem cells to migrate. In one embodiment, the treatments for migrating hematopoietic stem cells include, but are not limited to, inhibition of CXCR4 (Future Oncol. 2007 Feb; 3(1):19-27), inhibition of epidermal growth factor receptor (EGFR) (Nature Medicine volume 16, pages 1141-1146 (2010)), induction of granulocyte colony stimulating factor (G-CSF) stimulation (Blood. 1995 Dec 15; 86(12): 4437-45), and induction of CXCR2 stimulation (Cell. 2018 Jan 11; 172 (1-2): 191-204.e10).

In one embodiment, the migration in the therapy and/or prevention of the present disclosure can be performed for abnormal stem cells having any of the features described herein using migration agents. Those skilled in the art can appropriately select a migration agent that can be used in migration in the therapy and/or prevention of the present disclosure. For example, before and after administration of the migration agent, those skilled in the art can select a migration agent that can be used for the therapy and/or prevention of the present disclosure by measuring the ratio of stem cells (e.g., abnormal stem cells having the features described herein) in bone marrow and/or blood and examining the migratory effect. In one embodiment, the migration in the therapy and/or prevention of the present disclosure can be performed for hematopoietic stem cells using migration agents. In one embodiment, the migration agents for hematopoietic stem cells may be, but are not limited to, migration agents having functions, such as inhibition of CXCR4 (e.g., Plerixafor (AMD3100)), inhibition of epidermal growth factor receptor (EGFR) (e.g., Gefitinib, Erlotinib, Afatinib, Osimertinib, and the like), induction of granulocyte colony stimulating factor (G-CSF) stimulation (e.g., Filgrastim, Nartograstim, Lenograstim, Pegfilgrastim and the like), and induction of CXCR2 stimulation (e.g., GROβ (MIP2)). In one embodiment, migration agents for hematopoietic stem cells can be used in any combination, which may be a combination of, for example, an inhibiting agent for CXCR4 (e.g., Plerixafor (AMD3100)) and a CXCR2 stimulation inducing agent (e.g., GROβ (MIP2)).

In one embodiment, suppression of the abnormal stem cell described herein can be performed by targeting any feature of the abnormal stem cell described herein and by causing to migrate and suppressing a target cell. For example, when a molecule (such as CD106, CD34, TNF-α, proinsulin, c-Kit, or Sca-1) that the abnormal stem cell described herein expresses on the cell surface is targeted, a molecule (such as antibody, T cell receptor, or small molecule compound) specifically binding to the molecule expressed on the cell surface can be used. When a cell that simultaneously expresses a plurality of types of molecules is targeted, for example, a molecule binding to a plurality of types of molecules such as multi-specific antibodies can be used to target a cell of interest.

In one embodiment, the suppression in the therapy and/or prevention of the present disclosure can be performed by causing to migrate and suppressing an abnormal stem cell characterized by at least one of CD106 abnormal expression, CD34 abnormal expression, TNF-α abnormal expression, proinsulin abnormal expression, and histone deacetylases (HDACs) abnormal expression. In one embodiment, the suppression in the therapy and/or prevention of the present disclosure can be performed by causing to migrate and suppressing an abnormal stem cell characterized by CD106 abnormal expression. In one embodiment, the suppression in the therapy and/or prevention of the present disclosure can be performed by causing to migrate and suppressing an abnormal stem cell characterized by (a) CD106 abnormal expression and (b) at least one of CD34 abnormal expression, TNF-α abnormal expression, proinsulin abnormal expression, and histone deacetylases (HDACs) abnormal expression.

In one embodiment, the suppression in the therapy and/or prevention of the present disclosure can be performed by using one or more suppressing agent which target at least one of CD106, CD34, TNF-α, proinsulin, and histone deacetylases (HDACs). In one embodiment, the suppression in the therapy and/or prevention of the present disclosure can be performed using a suppressing agent which targets CD106. In one embodiment, the suppression in the therapy and/or prevention of the present disclosure can be performed using one or more suppressing agents which target (a) CD106 and (b) at least one of CD34, TNF-α, proinsulin, and histone deacetylases (HDACs). Any suppressing agent described herein can be used as a suppressing agent, which is, for example, an antibody binding to the molecule described above. In one embodiment, a suppressing agent which targets histone deacetylases (HDACs) can be an HDAC inhibiting agent. Examples thereof include, but are not limited to, TSA (trichostatin A), VPA (valproic acid), sodium butyrate (NaBu), SAHA (suberoylanilide hydroxamic acid or vorinostat), sodium phenylbutyrate, depsipeptide (FR901228, FK228), trapoxin (TPX), cyclic hydroxamic acid-containing peptide 1 (CHAP1), MS-275, LBH589, PXD101, and the like.

In one embodiment, the therapy and/or prevention of the present disclosure can be therapy and/or prevention of diabetes mellitus. In one embodiment, the therapy and/or prevention of the present disclosure can be therapy and/or prevention of neuropathy, nephropathy, hepatopathy, retinopathy, fatty liver, gastrointestinal disorder, delayed bone fracture healing, eating disorder, or dermatopathy.

The therapy and/or prevention of the present disclosure may be combined with any known treatment or means for therapy and/or prevention (e.g., treatment or means for therapy and/or prevention of diabetes mellitus and/or a disease, disorder, and/or symptom associated with diabetes mellitus).

### (Diagnosis of diabetes mellitus and/or a disease, disorder, and/or symptom associated with diabetes mellitus or a risk thereof based on the migration and/or residual state of an abnormal stem cell)

In one aspect, the present disclosure provides diagnosis of diabetes mellitus and/or a disease, disorder, and/or symptom associated with diabetes mellitus or a risk thereof based on the migration and/or residual state of the abnormal stem cell described herein. This diagnosis can be accomplished by any means such as performing a method or providing a composition, combination, kit, or system for the objective.

In one embodiment, the diagnosis of the present disclosure can be performed by detecting, at a specific site, the presence and/or abundance of at least some cells having at least one feature of the abnormal stem cell described herein. As used herein, unless otherwise noted, the "abundance" of cells means not only the number of cells (or any indicator of the number of cells), but also the proportion of specific cells in the cell population (or any indicator of the proportion of specific cells). In one embodiment, the diagnosis of the present disclosure can be performed by detecting the presence and/or abundance of the abnormal stem cells described herein in the bone marrow and/or bone marrow niche. In one embodiment, the diagnosis of the present disclosure can be performed by detecting the presence and/or abundance of the abnormal stem cells described herein in the circulating blood. In one embodiment, the diagnosis of the present disclosure can be performed by detecting the presence and/or abundance of abnormal stem cells at a specific site in a subject administered with the migration agent described herein, which can be performed, for example, based on the change in the presence and/or abundance of the abnormal stem cells at a specific site (e.g., bone marrow) before and after the administration of the migration agent. In this embodiment, for example, if the abundance of abnormal stem cells in a subject's bone marrow decreases after administration of a migration agent, the subject may be diagnosed with diabetes mellitus and/or a disease, disorder, and/or symptom associated with diabetes mellitus or a risk thereof; and in one embodiment, this subject may be subjected to the treatment for the therapy and/or prevention of the present disclosure.

In one embodiment, the diagnosis of the present disclosure can be performed based on a quantitative indicator (e.g., the amount of expression of cell surface protein or the amount of mRNA expression) of at least one feature of the abnormal stem cell described herein. In one embodiment, a subject can be determined to have diabetes mellitus and/or a disease, disorder, and/or symptom associated with diabetes mellitus or a risk thereof, or can be determined to be suitable for the abnormal cell migration treatment of the present disclosure when a sample (e.g., blood sample or bone marrow sample) derived from the subject or a hematopoietic stem cell (e.g., CD34 positive cell) contained in the sample exhibits the amount of expression of mRNA of CD106 which is higher than that of a corresponding sample from a population of non-diabetic subjects or a hematopoietic stem cell (e.g., CD34 positive cell) contained in the sample by 10% or greater, 20% or greater, 30% or greater, 40% or greater, 50% or greater, 60% or greater, 70% or greater, 80% or greater, 90% or greater, 100% or greater, 150% or greater, or 200% or greater.

In one embodiment, the diagnosis of the present disclosure can be performed based on the ratio of cells exhibiting at least one feature (including a quantitative indicator) of the abnormal stem cell described herein. In one embodiment, the diagnosis of the present disclosure can be performed based on the ratio of cells exhibiting CD106 expression at a level that is not normal among hematopoietic stem cells of a subject, and optionally, the diagnosis of the present disclosure can be performed by comparison with the ratio of cells exhibiting CD106 expression at a level that is not normal among hematopoietic stem cells in a population of non-diabetic subjects or a population of a diabetic subjects. In one embodiment, a subject can be determined to have diabetes mellitus and/or a disease, disorder, and/or symptom associated with diabetes mellitus or a risk thereof, or can be determined to be suitable for the abnormal cell migration treatment of the present disclosure when the ratio of cells exhibiting CD106 expression at a level that is not normal among hematopoietic stem cells at a specific site (e.g., bone marrow) in the subject is 1.1-fold or greater, 1.2-fold or greater, 1.3-fold or greater, 1.4-fold or greater, 1.5-fold or greater, 1.6-fold or greater, 1.7-fold or greater, 1.8-fold or greater, 1.9-fold or greater, 2-fold or greater, 2.1-fold or greater, 2.2-fold or greater, 2.3-fold or greater, 2.4-fold or greater, 2.5-fold or greater, 2.6-fold or greater, 2.7-fold or greater, 2.8-fold or greater, 2.9-fold or greater, 3-fold or greater, 3.5-fold or greater, 4-fold or greater, 4.5-fold or greater, or 5-fold or greater the ratio of cells exhibiting CD106 expression at a level that is not normal to hematopoietic stem cells in a population of non-diabetic subjects.

In one embodiment, the diagnosis of the present disclosure can be performed by detecting any feature of the abnormal stem cell described herein. In one embodiment, the diagnosis of the present disclosure can be performed by detecting a cell which has at least one feature of CD106 abnormal expression, CD34 abnormal expression, TNF-α abnormal expression, proinsulin abnormal expression, and histone deacetylases (HDACs) abnormal expression, and optionally further has other features (e.g., being c-Kit positive, being Sca-1 positive, being hematopoietic stem cell lineage marker negative, features of a short-term hematopoietic stem cell described above (such as being CD38 negative)) of the abnormal stem cell described herein. In one embodiment, the diagnosis of the present disclosure can be performed by detecting a cell characterized by CD106 abnormal expression. In one embodiment, the diagnosis of the present disclosure can be performed by detecting a cell which is characterized by (a) CD106 abnormal expression and (b) at least one of CD34 abnormal expression, TNF-α abnormal expression, proinsulin abnormal expression, and histone deacetylases (HDACs) abnormal expression, and optionally further has other features (e.g., being c-Kit positive, being Sca-1 positive, being hematopoietic stem cell lineage marker negative, features of a short-term hematopoietic stem cell described above (such as being CD38 negative)) of the abnormal stem cell described herein.

In one embodiment, the diagnosis of the present disclosure can be performed by using one or more detection agents which target at least one of CD106, CD34, TNF-α, proinsulin, and histone deacetylases (HDACs). In one embodiment, the diagnosis of the present disclosure can be performed by using a detection agent which targets CD106. In one embodiment, the diagnosis of the present disclosure can be performed by using one or more detection agents which target (a) CD106 and (b) at least one of CD34, TNF-α, proinsulin, and histone deacetylases (HDACs). Any detection agent described herein can be used as a detection agent, which includes, for example, an antibody binding to the molecule described above.

In one embodiment, the diagnosis of the present disclosure may be performed by administering a detection agent to a subject, or may be performed by testing a sample derived from a subject. For example, the diagnosis of the present disclosure can be performed by administering the detection agent of the present disclosure to a subject to detect the presence and/or abundance of the abnormal stem cells of the present disclosure in the bone marrow (or a particular niche thereof). For example, the diagnosis of the present disclosure can be performed by obtaining a sample (e.g., blood sample or bone marrow sample) comprising a cell from a subject and confirming whether a cell having at least one feature of the abnormal stem cell of the present disclosure and/or a marker that indicates its presence in the bone marrow (or a particular niche thereof) is present.

In one embodiment, the diagnosis of the present disclosure can be diagnosis of diabetes mellitus. In one embodiment, the diagnosis of the present disclosure can be diagnosis of neuropathy, nephropathy, hepatopathy, retinopathy, fatty liver, gastrointestinal disorder, delayed bone fracture healing, eating disorder or dermatopathy, or a risk thereof.

The diagnosis of the present disclosure may be combined with any known diagnosis (e.g., diagnosis of diabetes mellitus and/or a disease, disorder, and/or symptom associated with diabetes mellitus).

In one embodiment, the therapy and/or prevention of the present disclosure can be performed based on the diagnosis of the present disclosure (e.g., on a subject who is expected to have an abnormal stem cell in the bone marrow as a result of the diagnosis of the present disclosure).

The therapy, prevention, and/or diagnosis of the present disclosure can be performed on any subject. In one embodiment, a subject is a mammal (e.g., a human, mouse, guinea pig, hamster, rat, mouse, rabbit, pig, sheep, goat, cow, horse, cat, dog, marmoset, monkey, chimpanzee, or the like). In a specific embodiment, a subject is a human.

### (Medicine, dosage form, and the like)

The suppressing agent and migration agent of an abnormal stem cell and the detection agent for an abnormal stem cell described herein can be provided as a composition or medicament in various forms.

It is preferable that an administration route which is effective in therapy, prevention, or detection of diabetes mellitus and/or a disease, disorder, and/or symptom associated with diabetes mellitus is used for the suppressing agent and migration agent of an abnormal stem cell and the detection agent for an abnormal stem cell described herein. For example, the administration route may be intravenous, subcutaneous, intramuscular, intraperitoneal, oral administration or the like. The mode of administration may be, for example, injection, capsule, tablet, granule or the like. An aqueous solution for injection may be stored, for example, in a vial or a stainless streel container. Further, an aqueous solution for injection may contain, for example, saline, saccharide (e.g., trehalose), NaCl, NaOH or the like. Further, a therapeutic drug may contain, for example, a buffer (e.g., phosphate buffer), stabilizer or the like.

The composition, medicament, suppressing agent, migration agent, detection agent and the like of the present disclosure generally comprise a therapeutically effective amount of active ingredient or a detectable amount of a detection agent and a pharmaceutically acceptable carrier or excipient. As used herein, "pharmaceutically acceptable" means government regulatory agency-approved or pharmacopoeia or other commonly recognized pharmacopoeia-listed for use in animals and more specifically in humans. As used herein, a "carrier" refers to a diluent, adjuvant, excipient or vehicle administered in conjunction with a therapeutic agent or detection agent. Such a carrier can be an aseptic liquid such as water or oil, including but not limited to liquids derived from petroleum, animal, plant or synthesis, as well as peanut oil, soybean oil, mineral oil, sesame oil and the like. When a medicament is orally administered, water is a preferred carrier. For intravenous administration of a pharmaceutical composition, saline and aqueous dextrose are preferred carriers. Preferably, aqueous saline solution and aqueous dextrose and glycerol solution are used as a liquid carrier for an injectable solution. Suitable excipients include light anhydrous silicic acid, crystalline cellulose, mannitol, starch, glucose, lactose, sucrose, gelatin, malt, rice, wheat flour, chalk, silica gel, sodium stearate, glyceryl monostearate, talc, sodium chloride, powdered skim milk, glycerol, propylene, glycol, water, ethanol, carmellose calcium, carmellose sodium, hydroxypropyl cellulose, hydroxypropyl methylcellulose, polyvinyl acetal diethylamino acetate, polyvinylpyrrolidone, gelatin, medium-chain fatty acid triglyceride, polyoxyethylene hydrogenated castor oil 60, saccharose, carboxymethylcellulose, corn starch, inorganic salt and the like. When desired, the composition can contain a small amount of wetting agent or emulsifier or pH buffer. These compositions can be in a form of solution, suspension, emulsion, tablet, pill, capsule, powder, sustained release mixture or the like. It is also possible to use traditional binding agents and carriers, such as tryglyceride, to prepare a composition as a suppository. Oral preparation can also comprise a standard carrier such as medicine grade mannitol, lactose, starch, magnesium stearate, sodium saccharin, cellulose, or magnesium carbonate. Examples of a suitable carrier are described in E. W. Martin, Remington's Pharmaceutical Sciences (Mark Publishing Company, Easton, U.S.A). Such a composition contains a therapeutically effective amount of therapy agent and preferably in a purified form, together with a suitable amount of carrier, such that the composition is provided in a form suitable for administration to a patient. A preparation must be suitable for the administration format. In addition, the composition may comprise, for example, a surfactant, excipient, coloring agent, flavoring agent, preservative, stabilizer, buffer, suspension, isotonizing agent, binding agent, disintegrant, lubricant, fluidity improving agent, corrigent or the like.

Examples of "salt" in one embodiment of the present disclosure include anionic salts formed with any acidic (e.g., carboxyl) group and cationic salts formed with any basic (e.g., amino) group. Salts include inorganic salts and organic salts, as well as salts described in, for example, Berge et al., J.Pharm.Sci., 1977, 66, 1-19. Further examples include metal salts, ammonium salts, salts with organic base, salts with inorganic acid, salts with organic acid and the like. "Solvent" in one embodiment of the present disclosure is a compound formed with a solute or solvent. For example, J.Honig et al., The Van Nostrand Chemist's Dictionary P650 (1953) can be referred for solvates. When a solvent is water, a solvate formed is a hydrate. It is preferable that the solvent does not obstruct the biological activity of the solute. Examples of such a preferred solvent include, but not particularly limited to water and various buffers.

In the present disclosure, when a medicament is administered, various delivery systems can be used together, and such systems can be used to administer the suppressing agent and/or migration agent of the present disclosure to a suitable site. Such a system, for example, can use encapsulation in liposomes, microparticles and microcapsules, endocytosis mediated by a receptor, construction of a therapeutic nucleic acid as a part of a retrovirus vector or other vector or the like. The method of introduction includes, but not limited to, intradermal, intramuscular, intraperitoneal, intravenous, subcutaneous, intranasal, epidural and oral routes. A medicament can be administered by any suitable route, such as by injection, bolus injection, or by absorption through epithelial or mucocutaneous lining (e.g., oral cavity, rectum, intestinal mucosa or the like). In addition, an inhaler or mistifier using an aerosolizing agent can be used as needed. Moreover, other biological active agents can also be administered together. Administration can be systemic or local.

In a preferred embodiment, a composition can be prepared as a pharmaceutical composition adapted for administration to humans in accordance with a known method. Such a composition can be administered by an injection. A composition for injection administration is typically a solution in an aseptic isotonic aqueous buffer. A composition can also comprise a local anesthetic such as lidocaine which alleviates the pain at the site of injection and a solubilizing agent as needed. Generally, ingredients can be supplied separately or by mixing the ingredients together in a unit dosing form and supplied, for example, in a sealed container such as an ampoule or sachet showing the amount of active agent or as a lyophilized powder or water-free concentrate. When a composition is to be administered by injection, the composition can be distributed by using an injection bottle containing aseptic agent-grade water or saline. When a composition is to be administered by injection, an aseptic water or saline ampoule for injection can also be provided such that the ingredients can be mixed prior to administration.

The composition, medicament, migration agent and suppressing agent of the present disclosure can be prepared as a neutral or salt form or other prodrugs (e.g., ester or the like). Pharmaceutically acceptable salts include salts formed with hydrochloric acid, phosphoric acid, a free carboxyl group derived from acetic acid, oxalic acid, tartaric acid or the like, salts formed with a free amine group, derived from isopropylamine, triethylamine, 2-ethylaminoethanol, histidine, procaine or the like, and salts derived from sodium, potassium, ammonium, calcium, or ferric hydroxide or the like.

The amount of the suppressing agent and/or migration agent of the present disclosure may vary depending on the properties of the disorder or condition. However, such an amount can be determined by those skilled in the art by a standard clinical technique based on the descriptions herein. Furthermore, an in vitro assay can be used in some cases to assist the identification of the optimal dosing range. The precise dose to be used in a preparation may also vary depending on the administration route or the severity of the disease or disorder. Thus, the dose should be determined in accordance with the judgment of the attending physician or the condition of each patient. The dosage is not particularly limited, but may be, for example, 0.001, 1, 5, 10, 15, 100 or 1000 mg/kg body weight per dosage or within a range between any two values described above. The dosing interval is not particularly limited, but may be, for example, 1 or 2 administration every 1, 7, 14, 21, or 28 days or 1 or 2 administrations in the range of period between any two values described above. The dosage, dosing interval, and dosing method may be appropriately selected depending on the age, weight, symptom, target organ or the like of the patient. Further, it is preferable that a therapeutic drug contains a therapeutically effective amount, or an amount effective for exerting a desired effect, of active ingredients. The effective dose can be estimated from a dose-response curve obtained from in vitro or animal model testing systems.

The pharmaceutical composition, therapeutic agent, or prevention agent of the present disclosure can be provided as a kit.

In a specific embodiment, the present disclosure provides an agent pack or kit comprising one or more containers filled with one or more components of the composition or medicament of the present disclosure. Optionally, information indicating approval of manufacture, use, or sale for administration to humans by a government agency regulating the manufacture, use or sale of medicaments or biological products in a stipulated form can be appended to such a container.

The formulation procedure for the therapeutic drug, prophylactic drug, or the like of the present disclosure as a medicament or the like is known in the art. The procedure is described, for example, in the Japanese Pharmacopoeia, the United States Pharmacopeia, pharmacopeia of other countries, or the like. Thus, those skilled in the art can determine the embodiment, such as the amount to be used, without undue experimentation from the descriptions herein.

As used herein, "or" is used when "at least one or more" of the listed matters in the sentence can be employed. When explicitly described herein as "within the range of two values", the range also includes the two values themselves.

Reference literatures such as scientific literatures, patents, and patent applications cited herein are incorporated herein by reference to the same extent that the entirety of each document is specifically described.

As described above, the present disclosure has been described while showing preferred embodiments to facilitate understanding. The present disclosure is described hereinafter based on Examples. The above descriptions and the following Examples are not provided to limit the present disclosure, but for the sole purpose of exemplification. Thus, the scope of the present invention is not limited to the embodiments and Examples specifically described herein and is limited only by the scope of claims.

### [Examples]

If necessary, an animal used in the following Example is handled in accordance with the animal experiments guidelines of Shiga University of Medical Science. For reagents, the specific products described in the Examples were used. However, the reagents can be substituted with an equivalent product from another manufacturer (Sigma-Aldrich, Wako Pure Chemical, Nacalai Tesque, R & D Systems, USCN Life Science INC, or the like).

In the following Examples, the abbreviations each have the following meaning.
- DM: Diabetes mellitus
- STZ-DM: Streptozotocin-induced diabetes mellitus
- KSL cell: c-Kit positive, Sca-1 positive, and Lineage (lineage marker) negative cell
- SNCV: Sensory nerve conduction velocity

### (Example 1: Identification of an abnormal stem cell involved in diabetes mellitus)

Stem cells obtained from a diabetes mellitus model mouse were analyzed to identify an abnormal cell involved in diabetes mellitus.

### Method

### Obtaining mice and bone marrow cells

C57BL/6J mice (wild-type, CLEA Japan, Inc., Osaka) were used for the test. Diabetes mellitus was induced by an intravenous injection of streptozotocin (STZ) (150 mg/kg) (Nacalai Tesque, Kyoto) to create type 2 diabetes mellitus models (STZ mice). Bone marrow cells were isolated from the mice that were 8 weeks old.

### FACS

Mononuclear cells were isolated from the whole bone marrow by using Ficoll-Paque Plus (GE Healthcare BioSciences AB, Uppsala, Sweden).

In order to evaluate expression of TNF-α and CD106, mononuclear cells were subjected to an immune reaction with a PE-Cy7-conjugated streptavidin antibody (BD Biosciences, San Jose, CA), biotin mouse lineage panel staining (BD Biosciences), an APC-conjugated anti-c-kit antibody (BD Biosciences), an APC-Cy7-conjugated anti-Ly6A/E antibody (Sca-1) (BD Biosciences), a fluorescein isothiocyanate (FITC)-conjugated anti-CD106 antibody (BD Biosciences), and a phycoerythrin (PE)-conjugated anti-TNF-α antibody (eBiosciences).

In order to evaluate proinsulin expression, mononuclear cells were reacted with a PE-Cy7-conjugated streptavidin antibody, an APC-conjugated anti-c-kit antibody, an APC-Cy7-conjugated anti-Ly6A/E antibody (Sca-1), and a biotin mouse lineage panel, followed by being fixed with BD Cytofix/CytoPerm (BD Biosciences). Next, a rabbit anti-insulin monoclonal antibody (Cell signaling technology) and a PE-conjugated anti-rabbit IgG antibody (Cell signaling technology) were applied to the mononuclear cells. Prior to the staining, a LIVE/DEAD fixable dead cell blue stain kit (ThermoFisher Scientific Inc., Waltham, Massachusetts, the US) was used to deplete dead cells.

After 4 hours from the staining, the cells were analyzed with FACS Canto II by using FACS DIVA software (BD Biosciences).

### Result

While proinsulin positive cells were found in a KSL fraction of mononuclear cells derived from the STZ-DM mice, no proinsulin positive cell was observed in a KSL fraction of the non-DM mice (Figure **1**). Similarly, while TNF-α positive cells were found in a KSL fraction of mononuclear cells derived from the STZ-DM mice, no TNF-α positive cell was observed in a KSL fraction of the non-DM mice (Figure **2**). CD106 positive cells were found in KSL fractions of mononuclear cells of both the STZ-DM mice and the non-DM mice, but more cells were expressed in the STZ-DM mice (Figure **3**).

Diabetic mice have TNF-α positive cells and proinsulin positive cells in KSL cells as well as increased CD106 expressing cells. It is considered that hematopoietic stem cells having these features cause the appearance of the feature of diabetes mellitus as a chronic disease.

### (Example 2: Identification of an abnormal stem cell involved in type 1 diabetes mellitus)

For stem cells obtained from type 1 diabetes mellitus model mice, an abnormal cell involved in diabetes mellitus was characterized in the same manner as Example 1.

### Method

### Obtaining mice and bone marrow cells

ICR mice (CLEA Japan, Inc., Osaka) and NOD mice (CLEA Japan, Inc., Osaka) were used for the test. Mononuclear cells were isolated in the same manner as Example 1.

In the same manner as Example 1, mononuclear cells were stained for TNF-α, CD106, c-kit, Sca-1, and mouse lineage, and FACS analysis was performed.

### Result

The ratio of KSL cells in the NOD mice decreased to about 25% as compared to the ICR mice (Figure **4**). The ratio of TNF-α positive cells and CD106 expressing cells included in a KSL cell population significantly increased in the NOD mice as compared to the ICR mice (Figure **5**).

TNF-α positive cells and CD106 expressing cells also increased in KSL cells of mice with type 1 diabetes mellitus. It is considered that hematopoietic stem cells having these features cause the appearance of the feature of diabetes mellitus as a chronic disease.

### (Example 3: Cell stage of an abnormal stem cell involved in diabetes mellitus)

A test was conducted to study at what stage of hematopoietic stem cell the abnormal stem cell is.

### Method

A side population (SP) of KSL cells was analyzed by FACS in accordance with the method reported by Goodell (see J Exp Med. 1996 Apr 1; 183(4):1797-806; Nat Med. 1997 Dec; 3(12):1337-45). Hoechest 33342 dye, which is cell permeable and binds to DNA, can be efficiently flowed out extracellularly from a hematopoietic stem cell. It has been reported that when this property is utilized and bone marrow cells stained with the dye are excited with ultraviolet light (350 nm) and developed with two types of optical filters, Hoechst blue and Hoechst red, a hematopoietic stem cell fraction is included in an unstained cell population (SP cell). Whole bone marrow cells were stained for 90 minutes precisely at 37°C by using Hoechest 33342 (Sigma-Aldrich Japan K.K., Tokyo, Japan) as a dye. Hoechest 33342 positive cells were incubated with verapamil hydrochloride (Tocris bioscience, Bristol, UK) for setting a gate, and mononuclear cells were isolated by Ficoll-Paque Plus. Next, the cells were stained with a biotin mouse lineage panel and then incubated with PE-Cy7-conjugated streptavidin, APC-conjugated anti-c-kit, APC-Cy7-conjugated anti-Ly6A/E (Sca-1), and PE-conjugated anti-TNF-α or FITC-conjugated anti-CD106. In order to deplete dead cells, propidium iodide (Sigma-Aldrich) was added to the sample to remove dead cells, and analysis was performed with FACS Aria Fusion using FACS DIV software (BD Biosciences). Cells stained with Hoechest 33342 were excited with ultraviolet light of 350 nm, and two types of optical filters, 450BP20 (450/20 nm band-pass filter) (Hoechst blue) and 675EFLP (675 nm long-pass edge filter) (Hoechst red), were used.

### Result

The ratio of SP cells in KSL cells increased and the ratio of non-SP cells decreased in STZ-DM mice as compared to non-DM mice (Figure **6**). Neither CD106 positive cell nor TNF-α positive cell was detected in SP cells of both the non-DM mice and the STZ-DM mice (Figures **7b** and **d**). On the other hand, among non-SP cells, although no TNF-α positive cell was detected from non-DM, TNF-α positive cells were detected from STZ-DM, and more CD106 positive cells were observed in the STZ-DM mice as compared to the non-DM mice (Figures **7a** and **c**). Thus, CD106 positive cells and TNF-α positive cells are present in a non-SP fraction of KSL cells in STZ-DM.

While CD106 positive cells and TNF-α positive cells, which characterize the abnormality of hematopoietic stem cells, were included in non-SP cells (short-term hematopoietic stem cell: ST-HSC) in KSL cells, CD106 positive cells and TNF-α positive cells were not included in SP cells (long-term hematopoietic stem cell: LT-HSC) in KSL cells at an earlier differentiation stage. This can suggest that the HSC abnormality in diabetes mellitus remains at the stage of progenitor cells among stem cells, no abnormality occurred in so-called "stem cells among stem cells", and it is possible to treat diabetes mellitus and an associated disease thereof by removing progenitor cells. While bone marrow transplantation would be required if "stem cells among stem cells" are a target of therapy, it is considered that drug therapy is possible if therapy is possible by removing progenitor cells.

In this manner, it was revealed that abnormal stem cells found in diabetes mellitus can be present in ST-HSCs.

### (Example 3: Further characterization of an abnormal stem cell)

An abnormal stem cell involved in diabetes mellitus was further characterized.

### Method

RNA was extracted from KSL cells obtained by FACS sorting on non-DM mice and STZ-DM mice in the same manner as Example 1, and the gene expression of histone deacetylase genes (Hdacs) was analyzed using QT-PCR.

### Result

Figure **8** shows the result. It was revealed that the mRNA expression of Hdac3, Hdac4, Hdac8, and Hdac9 significantly increased in the STZ-DM mice as compared to the non-DM mice.

Since diabetic KSL cells showed increased expression of an epigenome-associated gene (Hdacs) that is important for regulating gene expression by a histone modification or the like, it was considered that hyperglycemia imparts an abnormal property to stem cells at a gene level. It has been reported that once cells were exposed to transient hyperglycemia, even if the cells are again subjected to an environment of a normal blood glucose level, the abnormality in the cells which was caused due to hyperglycemia is maintained (El-Osta et.al. J Exp Med. 2008 Sep 29; 205(10): 2409-17). This report also suggests that an increase in the expression of the histone deacetylase gene which was caused in KSL cells can be involved in appearance of CD106, TNF-α, and proinsulin positive cells. A possible way to reveal this point would be to study whether it is possible to perform therapy with an Hdac inhibiting agent (e.g., trichostatin A).

### (Example 5: Onset of diabetes mellitus due to an abnormal stem cell)

The inventors tested whether the abnormal stem cells that were found above are a cause of diabetes mellitus.

### Method

Figure **9** shows the outline of the test to transplant non-DM or STZ-DM KSL cells to a normoglycemic mouse. GFP-Tg mice (The Jackson Laboratory, Bar Harbor, ME) were subjected to STZ treatment (STZ-DM GFP) in the same manner as Example 1 or control treatment with an intravenous injection of a citrate buffer (non-DM GFP). After 3 months, KSL cells obtained from each of the non-DM mice and the STZ-DM mice were transplanted to normoglycemic wild-type C57BL/6J mice (CLEA Japan, Inc., Osaka) that had been irradiated with a lethal dose of radiation at 9 Gy (non-DM-derived KSL-T or STZ-DM-derived KSL-T, respectively) (Figure **10**, left). The blood glucose level after 3 months from the transplantation was observed (non-DM-derived KSL-T (n = 9), STZ-DM-derived KSL-T (n = 10)). SNCV in the sciatic nerve was measured after 3 months from the transplantation ((non-DM-derived KSL-T (n = 5), STZ-DM-derived KSL-T (n = 6)) (Figure **10**, right). A dorsal root ganglion (DRG) was obtained from each mouse after 3 months from the transplantation, and immunofluorescence staining was performed for a nucleus, GFP, MAP2, proinsulin, and TNF-α (PE-conjugated) (Figure **11**, Figure **12**, and Figure **13**).

The SNCV measurement described above was performed in the following procedure. A sensory nerve conduction velocity test was conducted using Medelec Sapphire EMG (Medelec, Woking, UK) in the mice under anesthesia (pentobarbital sodium, 5 mg/kg i.p.) at 30°C to 32°C. A skin was cut from the left back surface of the femur to expose the sciatic nerve. The sensory nerve action potential (SNAP) was recorded, and the sensory nerve conduction velocity (SNCV) was calculated by dividing the distance from the stimulated site to the recorder site by the early latency of the SNAP.

### Result

While the feature of abnormal stem cells such as TNF-α or proinsulin was observed in a KSL cell fraction of the STZ-DM-derived KSL-T mice, no feature of abnormal stem cells was observed in a KSL cell fraction of the non-DM-derived KSL-T mice.

As a result of transplantation of abnormal KSL cells found in a diabetic mouse into a normal mouse, a symptom similar to diabetic neuropathy was exhibited despite having a normal blood glucose level. This result revealed that abnormal stem cells are not eliminated even when the blood glucose level of a diabetic mouse is normalized. This finding indicated that abnormal cells settled in the bone marrow while being abnormal, in other words, the abnormal cells settled as pathological stem cells. Further, since it is understood that cells derived from the abnormal stem cells migrate to nerve tissue to develop neuropathy, it was revealed that diabetes mellitus will not be cured even when the blood glucose is controlled as long as said stem cells remain. Moreover, when the bone marrow comprising abnormal stem cells of the diabetic mouse was transplanted into a normal mouse, impaired glucose tolerance was observed, wherein glucose metabolism disorder that is the core of diabetes mellitus was also reproduced by the bone marrow transplantation.

This result is consistent with a clinical symptom that diabetes mellitus and a diabetic complication are not cured even when hyperglycemia is improved, suggesting that the ultimate therapy target for diabetes mellitus is an abnormal hematopoietic stem cell. In this manner, glucose metabolism disorder and diabetic neuropathy could be reproduced in a normal mouse by transplanting diabetic hematopoietic stem cells into the normal mouse.

### (Example 6: Feature of an abnormal stem cell in a human diabetic patient)

Patients in the intensive care unit of Shiga University of Medical Science Hospital were investigated. Subjects from whom samples were collected were patients diagnosed with diabetes mellitus (three patients) (three males/no female, average age of 72.0 ± 14.2) and healthy subjects (four subjects) (three males/one female, average age of 76.5 ± 8.5).

Blood samples were collected from these subjects, and RNA was extracted from the blood samples. Specifically, 1.5 ml of blood was collected, DNA was degraded using with a DNAse (Qiagen, Hilden, Germany), and RNA was then extracted by using a QIAamp RNA blood mini kit (Qiagen, Hilden, Germany). cDNA was created from the obtained RNA by using a SuperScript^{®} III First-Strand Synthesis System (Invitrogen/ThermoFisher scientific, MA, the US). Quantitative PCR was performed using a Power SYBR Green PCR Master Mix in a real-time PCR system (Applied Biosystems/ThermoFisher scientific, MA, the US), and the expressed mRNA was quantified. Comparison between the two groups was performed with a t-test. Figure 15 shows the result.

Similar to the results from mice, it was observed that the expression of insulin, TNF-α, and CD106 tended to increase in human diabetic patients. Further, it was observed that the expression of CD34 tended to increase in diabetic patients. Since CD34 represents a feature of a stem cell in humans, the above result suggests that an abnormal stem cell is also present in human diabetic patients, similar to the results from mice.

### (Example 7: Therapy of type 1 diabetes mellitus targeting an abnormal stem cell)

It is studied whether, when an insulin pellet is subcutaneously implanted to the back of NOD mice which were observed to have an increase in the blood glucose level to normalize the blood glucose level and an anti-CD106 antibody is then administered via tail vein administration at a dose of 250 µg/mouse every week, the normal blood glucose level can be maintained without relying on the insulin pellet.

Diabetic mice of which blood glucose level was almost normalized by implantation of an insulin pellet are irradiated with a radiation to kill bone marrow stem cells (both LT-HSCs and ST-HSCs), and only LT-HSCs collected from a normal mouse are transplanted to the bone marrow instead. With this operation, ST-HSCs will be newly produced from LT-HSCs in the bone marrow of the mice subjected to transplantation. In this case, since abnormal ST-HSCs that were originally present in the diabetic mice and T cell progenitor cells (abnormality is observed in STZ mice and NOD mice having diabetes mellitus) and B cell progenitor cells (abnormality is observed in NOD mice prior to the onset of diabetes mellitus) differentiated from the ST-HSCs are already dead, it is considered that these cells do not damage an organ. Further, similar diabetic mice are prepared, and similar LT-HSC bone marrow transplantation is performed while keeping the blood glucose level high without implanting an insulin pellet. Since hyperglycemia remains in these mice, it is expected that abnormal ST-HSCs are generated from LT-HSCs and that the ST-HSCs will damage various organs. Thus, it is expected that diabetes mellitus and a complication thereof do not heal.

Abnormal ST-HSCs are removed by treatment with insulin pellet + LT-HSCs, the impaired pancreatic islet is normalized, and the complication can be eliminated.

### (Example 8: Combination treatment of a suppressing agent and a migration agent for stem cells in streptozotocin-induced diabetes mellitus mice)

With streptozotocin-induced diabetic mice, the therapeutic effect of a combination of a suppressing agent and a migration agent on abnormal stem cells was tested.

### Method

Streptozotocin-induced diabetic mice (3 months after the onset of diabetes mellitus) prepared in the same manner as above were subcutaneously injected with AMD3100 (5 mg/kg: abcam) + GROβ (0.1 mg/kg: Peprotech) together with saline, and after 15 minutes, the mice were injected with anti-CD106 antibody (250 µg/mouse) via tail vein administration (GA-CD106 group). This treatment was performed weekly. Similarly, testing was conducted on a group to which anti-CD106 antibody was administered without administration of AMD3100 and GROβ (CD106 group), and on a group to which none of AMD3100, GROβ and anti-CD106 antibody was administered (non-treated group). Blood glucose levels and body weight were measured daily (Figure **15**), and the mice were perfused and fixed 20 days after the start of therapy.

After 24 hours of fixation, the fixed mice were transferred to 15% sucrose and 0.1M PB solution, and then, the pancreas was collected from the mice to prepare a frozen block of the pancreas. Thereafter, frozen sections of 8 µm were prepared with a cryostat.

Thereafter, the prepared frozen sections were immunohistochemically stained by performing the following procedure (Figure **16**).
- Wash three times with PBS (-) for ten minutes.
- Soak in a 0.3% H₂O₂ PBS (-) solution at room temperature for thirty minutes to inactivate the endogenous peroxidase.
- Wash three times with PBS (-) for five minutes.
- Incubate for one hour at room temperature with blocking buffer (5% normal goat serum in PBS + 0.3% triton X-100).
- Add a primary antibody (anti-insulin antibody: CST) and incubate at 4°C overnight.
- Wash three times with PBS (-) for five minutes.
- Add ImmPRESS Reagent (Anti-rabbit: VECTOR Laboratories) and incubate at room temperature for thirty minutes.
- Wash three times with PBS (-) for five minutes.
- Add ImMPACT DAB substrate (VECTOR Laboratories) and allow it to react at room temperature for thirty seconds.
- Add dH₂O to stop the DAB reaction.
- Counter-stain with hematoxylin for thirty seconds.
- Wash with tap water.
- Dehydrate and infiltrate.

The prepared section samples were observed about 8 to 10 islets randomly selected under a microscope, and the ratio of the size of insulin-positive area to the size of the islets was calculated by Image J software (Figure 17).

### Result

The group in which stem cells were caused to migrate and treated with the anti-CD106 antibody showed a decrease in blood glucose level (Figure **15**), and the group had an increased insulin-positive area in immunohistochemistry compared to the non-treated group (Figure **17**).

The suppression of abnormal stem cell after stem cell migration restored the insulin-positive area in the pancreatic islets, which remained only about 10% due to STZ administration, to about 30-40%. This indicates that abnormal stem cells released peripherally from the bone marrow niche by administration of the stem cell migration agent may manifest altered function by the anti-CD106 antibody, while normally functioning stem cells may regenerate islets.

### (Example 9: Combination treatment of a suppressing agent and a migration agent for stem cells in NOD mice)

With spontaneous type 1 diabetes model mice (NOD mice), the therapeutic effect of a combination of a suppressing agent and a migration agent for abnormal stem cells was tested.

### Method

NOD mice, which develop spontaneously type 1 diabetes models, were purchased from CLEA Japan, Inc. (Osaka), and the blood glucose level and body weight of the mice were measured every two weeks. For the mice in which their blood glucose levels increased, was observed, AMD3100 (5 mg/kg: abcam) + GROβ (0.1 mg/kg: Peprotech) was prepared with saline and then subcutaneously injected into them, and fifteen minutes later, the mice were administered anti-CD106 antibody (250 µg/mouse) via tail vein (administration interval was weekly). After the start of the therapy, the blood glucose level and body weight were measured daily (Figure **18**).

In addition, prior to the start of the therapy, in order to confirm the islet status of ICR mice, NOD mice that did not develop diabetes mellitus yet, and NOD mice that developed diabetes mellitus (all mice used were of the same age in week), perfusion fixation was performed, and immunostaining was performed as follows (Figure **19**).
- Wash three times with PBS (-) for ten minutes.
- Soak in a 0.3% H₂O₂ PBS (-) solution at room temperature for thirty minutes to inactivate the endogenous peroxidase.
- Wash three times with PBS (-) for five minutes.
- Incubate for one hour at room temperature with blocking buffer (5% normal goat serum in PBS + 0.3% triton X-100).
- Add a primary antibody (anti-insulin antibody: CST) and incubate at 4°C overnight.
- Wash three times with PBS (-) for five minutes.
- Add ImmPRESS Reagent (Anti-rabbit: VECTOR Laboratories) and incubate at room temperature for thirty minutes.
- Wash three times with PBS (-) for five minutes.
- Add ImMPACT DAB substrate (VECTOR Laboratories) and allow it to react at room temperature for thirty seconds.
- Add dH₂O to stop the DAB reaction.
- Counter-stain with hematoxylin for thirty seconds.
- Wash with tap water.
- Dehydrate and infiltrate.

Images of each of the prepared slides were acquired.

### Result

In the NOD mice, the islet inflammatory reaction was already very strong before the onset of diabetes mellitus and insulin staining was very low (Figure **19**). In addition, it was confirmed that the blood glucose level of the diabetes mellitus mice decreased by the above treatment (Figure **18**).

The combination therapy of the migration agent and the antibody showed a clear hypoglycemic effect. This effect is considered to be due to the restoration of pancreatic islet function by the antibody.

It has been suggested that removal of abnormal hematopoietic stem cells is also a useful therapeutic strategy for type 1 diabetes mellitus.

### (Example 10: Abnormal cell in the bone marrow of a human diabetic patient)

It was confirmed that the bone marrow of a human diabetic patient also has an abnormal cell.

The inventors studied the presence or absence of the appearance of a proinsulin positive cell in the bone marrow of patients with type 2 diabetes mellitus (DM) who were hospitalized in Shiga University of Medical Science and autopsied between January 1, 2000 and December 31, 2010. The tissue was embedded in paraffin in Shiga University of Medical Science, Anatomy Center. A 5 µm thick section of the paraffin-embedded sample was treated for immunohistochemistry by using avidin-biotin-peroxidase complex (ABC) method and diaminobenzidine (DAB)-nickel reaction. After the section was deparaffinized in xylene and alcohol, the section was treated with a microwave (for 10 minutes at 0.5 kW at a pH of 6.0 in 10 mmol/L of citrate buffer), followed by incubation overnight with an antibody against proinsulin (mouse monoclonal, Abcam, UK) diluted at 1:1,000 in 0.1% PBS comprising 0.3% Triton X-100 (PBST). Subsequently, treatment for immunohistochemistry was performed at 4°C. After the DAB-nickel reaction, the segment was counterstained with a nuclear fast red solution.

Figure **20** shows the result. While proinsulin expression was not observed in bone marrow cells derived from patients without DM, proinsulin expression was observed in bone marrow cells derived from patients with DM. It is considered that the finding on an abnormal stem cell observed in mice is also applicable to humans.

### (Example 11: Application to humans)

A bone marrow-derived abnormal hematopoietic stem cell is identified in a human diabetic patient, and diabetes mellitus is treated while targeting the abnormal hematopoietic stem cell.

Research Plan 1: Identification of a bone marrow-derived abnormal hematopoietic stem cell in a diabetic patient

### (Subject)

For a non-diabetic group, volunteers who have no prior history of impaired glucose tolerance and are not currently receiving therapy for diabetes mellitus are recruited from the staff of Shiga University of Medical Science and Shiga University of Medical Science Hospital. The blood glucose level and HbA1c are continuously measured, and those who satisfy casual blood glucose level < 140 mg/dl and HbA1c < 6.0% are defined as non-diabetes melitus. 20 people are registered as a control group. Those who have HbA1c that is 6.5% or greater or who are under therapy for diabetes mellitus are defined as a diabetic group. A list of patients whose sex and age are matched with the non-diabetic group is prepared based on the electronic medical record from the patients regularly attending the outpatient clinic of diabetes mellitus and endocrine internal medicine of Shiga University of Medical Science Hospital. 80 patients (40 patients with type 1 diabetes mellitus and 40 patients with type 2 diabetes mellitus) are registered at random.

### (Research method)

Medical questions are asked to the subjects, the height and body weight of the subjects are measured, a blood test and a urine test are performed, and the presence or absence of diabetes mellitus is determined. Mononuclear cells are extracted from the collected blood, CD34-labelled bone marrow progenitor cells are collected and fixed, and the form and expressed protein are identified by immunostaining. Further, after mRNA is extracted, cDNA is prepared and the amount of expression of mRNA is quantified by quantitative PCR. Specifically, the amount of expression of TNF-α mRNA and insulin mRNA or the like is measured in CD34 positive and CD106 positive (CD34/CD106) bone marrow progenitor cells in peripheral blood. The presence or absence of expression of protein in these cells is also measured. Association between the presence or absence of a diabetic complication in the diabetic patients and blood glucose control is also analyzed. A nerve conduction velocity test, an electrocardiogram R-R interval test, an ophthalmoscopy, a urinary albumin excretion rate, quantification of the amount of intraperitoneal fat using abdominal CT, a blood lipid test, an electrocardiogram, a carotid artery echo test, and a lower limb artery echo test are performed to check the presence or absence of diabetic neuropathy, retinopathy, nephropathy, fatty liver, and dyslipidemia, which are representative complications, and macrovasculopathy.

### (Prediction of results)

(1) While expression of TNF-α mRNA and insulin mRNA is observed in CD34/CD106 bone marrow progenitor cells in peripheral blood of the non-diabetic group and the type 2 diabetic group, the amount of expression increases in the type 2 diabetic group. On the other hand, in the type 1 diabetic group, while expression of TNF-α mRNA increases in the CD34/CD106 bone marrow progenitor cells as compared to non-diabetes melitus, insulin mRNA is not expressed at all.
(2) There are very few cells expressing TNF-α protein and proinsulin protein in CD34/CD106 bone marrow progenitor cells in peripheral blood of the non-diabetic group. Meanwhile, cells expressing both of the proteins increase in type 2 diabetes mellitus. On the other hand, in type 1 diabetes mellitus, while cells expressing TNF-α protein increase, there is no cell expressing proinsulin.
(3) In the type 1 diabetic patients, onset of diabetic neuropathy, retinopathy, nephropathy, fatty liver, and dyslipidemia, which are representative complications, is associated with an increase in TNF-α protein positive cells in CD34/CD106 bone marrow progenitor cells in peripheral blood. On the other hand, in the type 2 diabetic patients, onset of diabetic neuropathy, retinopathy, nephropathy, fatty liver, and dyslipidemia is associated with an increase in TNF-α protein positive cells and an increase in proinsulin positive cells in CD3/CD106 bone marrow progenitor cells in peripheral blood.

### (Discussion and expectation of the conclusion)

1) Non-diabetes mellitus has CD34/CD106 bone marrow progenitor cells which express insulin mRNA and TNF-α mRNA, although only slightly, in blood. It is expected that these cells function as an endothelial cell which presents an autoantigen when homing to the pancreatic islet.
2) In type 2 diabetes mellitus, these cells (CD34/CD106 bone marrow progenitor cells expressing insulin mRNA and TNF-α mRNA) are present in the bone marrow and blood due to hyperglycemia. It is expected that these cells cause insulin resistance or various complications by prior expression of proinsulin and TNF-α protein and, concurrently, differentiation into a vascular endothelium with an abnormal function or possession of an abnormal cell fusion ability.

### Research Plan 2: Therapy of diabetes mellitus targeting a bone marrow-derived abnormal hematopoietic stem cell

### (Subject)

In accordance with Research Plan 1, 280 diabetic patients (140 patients with type 1 diabetes mellitus and 140 patients with type 2 diabetes mellitus) are registered in Shiga University of Medical Science and the collaborative research facility. It is believed that both type 1 diabetes mellitus and type 2 diabetes mellitus do not heal upon disease onset. However, it is known that the honeymoon phase, in which temporary remission is exhibited by strict blood glucose control using insulin, appears in type 1 diabetes mellitus. Although the period of the phase varies depending on the report, it has been reported that the period is generally 1 month to 13 years (Wallensteen M, Dahlquist G, Persson B, Landin-Olsson M, Lernmark A, Sundkvist G, Thalme B (1988) Factors influencing the magnitude, duration, and rate off all of β-cell function in type 1 (insulin-dependent) diabetic children followed for two years from their clinical diagnosis. Diabetologia 31: 664-669). Thus, it may be difficult to discern whether carrying out the present therapy plan has resulted in the honeymoon phase or healing of the disease itself. For type 2 diabetes mellitus, it has been reported that insulin resistance becomes mild by strict control (H.E. Lebovitz (2001) Insulin resistance: definition and consequences. Clin Endocrinol Diabetes 109 Suppl 2: S135-S148). This may result in improvement in the amount of a therapeutic drug such as insulin or oral agents as well as the endogenous insulin secretion ability. Thus, in the present therapy research, it is necessary to prepare a group to be treated by insulin alone and a group to be treated by a novel therapeutic method to compare and study these two groups for both type 1 and type 2.

### (Research method)

Medical questions are asked to the subjects, the height and body weight of the subjects are measured, a blood test and a urine test are performed, and the presence or absence of diabetes mellitus is determined. Mononuclear cells are extracted from the collected blood, CD34-labelled bone marrow progenitor cells are collected and fixed, and the form and expressed protein are identified by immunostaining. Further, after mRNA is extracted, cDNA is prepared and the amount of expression of mRNA is quantified by quantitative PCR. Specifically, the amount of expression of TNF-α mRNA and insulin mRNA or the like is measured in CD34 positive and CD106 positive bone marrow progenitor cells in peripheral blood. The presence or absence of expression of protein in these cells is also measured. Association between the presence or absence of a diabetic complication in the diabetic patients and blood glucose control is also analyzed. A nerve conduction velocity test, an electrocardiogram R-R interval test, an ophthalmoscopy, a urinary albumin excretion rate, quantification of the amount of intraperitoneal fat using abdominal CT, a blood lipid test, a carotid artery echo test, and a lower limb artery echo test are performed to check the presence or absence of diabetic neuropathy, retinopathy, nephropathy, fatty liver, and dyslipidemia, which are representative complications, and macrovasculopathy.

The type 1 cases are classified at random into seven groups each having 20 cases. The type 2 cases are classified at random into seven groups each having 20 cases. 20 cases of each of type 1 and type 2 are controlled for three months with insulin therapy alone (control group). For 60 cases among the remaining 120 cases, each of the following three types of therapies is started for 20 cases simultaneously with the start of insulin therapy (therapy group without the migration agent). 1) An anti-TNF-α antibody (40 mg/kg of adalimumab or 3 mg/kg of infliximab), 2) an anti-CD106 antibody (0.8 mg/kg), or 3) trichostatin (0.5 mg/kg) is intravenously administered once a week, and the therapy is continued for twelve weeks. For the remaining 60 cases, each of the following three types of therapies is started for 20 cases simultaneously with the start of insulin therapy (therapy group with the migration agent). 1) An anti-TNF-α antibody (40 mg/kg of adalimumab or 3 mg/kg of infliximab), 2) an anti-CD106 antibody (0.8 mg/kg), or 3) trichostatin (0.5 mg/kg) is intravenously administered together with the cell migration agents, Groβ (100 µg/kg) + Plerixafor (0.24 mg/kg), once a week, and the therapy is continued for twelve weeks.

### (Method for determining the therapeutic effect)

The patients perform self-monitoring of blood glucose 6 times a day. The target of blood glucose control is to achieve a pre-meal blood glucose level which is 140 mg/dl or less and a blood glucose level after 2 hours post-meal which is 200 mg/dl or less by insulin therapy. The amount of insulin is actively increased or decreased so as to satisfy the control criteria. Ultimately, the therapy research period ends with a dosage of insulin required for blood glucose control in week 12.

The therapeutic effect is determined by follow-up determination prior to the therapy, at the time of the end of the therapy, and after 3 months, 6 months, 9 months, and 12 months from the end of the therapy, considering the following determination items as the therapeutic effect.

### (Determination items)

### (A) Effect of eliminating an abnormal hematopoietic stem cell

Expressed protein (such as CD34, proinsulin, TNF-α, or CD106) and expressed gene (such as CD34 mRNA, insulin mRNA, TNF-α mRNA, or CD106 mRNA) in CD34/CD10 bone marrow progenitor cells in peripheral blood are quantified.

### (B) Effect of healing diabetes mellitus

Blood glucose level, HbA1c, urinary CPR, lipid, and insulin secretion ability with a glucagon loading test are measured prior to and after the therapy. A pancreatic islet-associated antibody is measured, and it is revealed whether the antibody is eliminated.

### (C) Therapeutic effect on a complication

The presence or absence and change of diabetic neuropathy, retinopathy, nephropathy, fatty liver, dyslipidemia, and macrovasculopathy, which are representative complications, are compared and studied prior to and after the therapy.

### (Prediction of results)

1) Therapy with insulin alone reveals the following.
   (A) Abnormal hematopoietic stem cells are not eliminated in both type 1 diabetes mellitus and type 2 diabetes mellitus.
   (B) The effect of healing diabetes mellitus is not observed.
   (C) The therapeutic effect on a complication is observed to some extent, but the complication does not heal.
2) Regardless of the presence or absence of a cell migration agent, novel therapy reveals the following.
   (A) Abnormal hematopoietic stem cells are eliminated in both type 1 diabetes mellitus and type 2 diabetes mellitus.
   (B) Both type 1 diabetes mellitus and type 2 diabetes mellitus heal once.
   (C) Progression of a complication is stopped and an obvious therapeutic effect is observed in both type 1 diabetes mellitus and type 2 diabetes mellitus.
3) Novel therapy with the addition of cell migration agents reveals the following.
   (A) Abnormal hematopoietic stem cells are eliminated earlier in both type 1 diabetes mellitus and type 2 diabetes mellitus by the novel therapy with a migration agent added thereto compared to the case without the migration agent.
   (B) The cure rate of both type 1 diabetes mellitus and type 2 diabetes mellitus is improved by the novel therapy with a migration agent added thereto compared to the case without the migration agent.
   (C) The cure rate of complications is improved in both type 1 diabetes mellitus and type 2 diabetes mellitus by the novel therapy with a migration agent added thereto compared to the case without the migration agent.

### (Discussion and expectation of the conclusion)

1) For type 1, it is possible that the blood glucose control effect results in the honeymoon phase even when diabetes mellitus is treated with insulin alone. However, in this case, the remission will eventually end, and there will be deficiency in the insulin secretion ability again.
2) For type 2 diabetes mellitus, it is possible that the blood glucose control is improved by therapy with insulin alone and therapy with insulin is no longer necessary. However, since it is not possible to eliminate the abnormal hematopoietic stem cells, diabetes mellitus does not heal.
3) Even when type 1 diabetes mellitus heals by the novel therapy with the addition of cell migration agents, there remains a possibility that autoimmunity, which was a cause of production of an autoantibody, may recur. However, the novel therapy with the addition of cell migration agents may be performed again.
4) Even when type 2 diabetes mellitus completely heals by the novel therapy with the addition of cell migration agents, abnormal hematopoietic stem cells may appear if hyperglycemia is recurred again due to an excessive intake of energy or a lack of exercise. Diabetes mellitus also can be treated in this case if the novel therapy with the addition of cell migration agents is performed again.

### (Note)

As described above, the present disclosure is exemplified by the use of its preferred embodiments. However, it is understood that the scope of the present invention should be interpreted solely based on the Claims. It is also understood that any patent, any patent application, and any references cited herein should be incorporated herein by reference in the same manner as the contents are specifically described herein.

The present application claims priority to Japanese Patent Application No. 2019-191369 filed to the Japan Patent Office on October 18, 2019. The entire content thereof is incorporated herein by reference.

### [Industrial Applicability]

The present disclosure provides improvements in the therapy for diabetes mellitus targeting stem cells, and based on this finding, provides new approaches for therapy and prevention of diabetes mellitus and/or a disease, disorder, and/or symptom associated with diabetes mellitus and diagnosis of diabetes mellitus and/or a disease, disorder, and/or symptom associated with diabetes mellitus or a risk thereof.

## Claims

1. A composition for use in treating and/or preventing diabetes mellitus and/or a disease, disorder, and/or symptom associated with diabetes mellitus, comprising a suppressing agent for an abnormal hematopoietic stem cell (HSC), **characterized in that** the composition is administered in combination with a stem cell migration agent.

2. The composition of claim 1, wherein the abnormal HSC is a cell in which a gene or protein selected from the group consisting of CD106 and a functional equivalent thereof is not expressed and/or does not function at a normal level.

3. The composition of claim 2, wherein the expression which is not at a normal level is overexpression.

4. The composition of any one of claims 1 to 3, wherein the suppressing agent comprises at least one selected from the group consisting of an anti-CD106 antibody or a functional variant thereof.

5. The composition of any one of claims 2 to 4, wherein the abnormal HSC is a cell in which a gene or protein selected from the group consisting of tumor necrosis factor alpha (TNF-α), histone deacetylase (HDAC), and proinsulin is further not expressed at a normal level.

6. The composition of any one of claims 1 to 5, wherein the suppressing agent comprises at least one selected from the group consisting of an anti-TNF-α antibody or a functional variant thereof and an HDAC inhibiting agent.

7. The composition of any one of claims 1 to 6, wherein the disease, disorder, and/or symptom comprises a diabetic complication.

8. The composition of any one of claims 1 to 6, wherein the disease, disorder, and/or symptom is selected from the group consisting of neuropathy, nephropathy, hepatopathy, retinopathy, fatty liver, gastrointestinal disorder, delayed bone fracture healing, eating disorder, and dermatopathy.

9. The composition of any one of claims 1 to 8, wherein the stem cell migration agent has an ability to cause the abnormal HSC to migrate from a niche.

10. The composition of any one of claims 1 to 9, wherein the stem cell migration agent comprises at least one agent selected from the group consisting of a CXCR4 antagonizing agent, a CXCR2 stimulating agent, an epidermal growth factor receptor (EGFR) inhibiting agent, and a granulocyte colony stimulating factor (G-CSF) agent.

11. The composition of any one of claims 1 to 10, wherein the stem cell migration agent comprises at least one selected from the group consisting of Plerixafor, GROβ2 (MIP2), Gefitinib, Erlotinib, Afatinib, Osimertinib, Filgrastim, Nartograstim, Lenograstim, and Pegfilgrastim.

12. A medicament for use in treating and/or preventing diabetes mellitus and/or a disease, disorder, and/or symptom associated with diabetes mellitus, the medicament comprising a combination of a suppressing agent for an abnormal hematopoietic stem cell (HSC) and a stem cell migration agent.

13. A composition for use in treating and/or preventing diabetes mellitus and/or a disease, disorder, and/or symptom associated with diabetes mellitus, the composition comprising a stem cell migration agent, **characterized in that** the composition is administered in combination with a suppressing agent for an abnormal hematopoietic stem cell (HSC) .

14. A composition for use in selecting a treatment for treating and/or preventing diabetes mellitus and/or a disease, disorder, and/or symptom associated with diabetes mellitus, the composition comprising an agent that detects a migration and/or residual state of an abnormal hematopoietic stem cell (HSC).

15. The composition of claim 14, wherein the migration and/or residual state is a migration from a niche of a bone marrow and/or a residual state at the niche of the bone marrow.

16. The composition of claim 14 or 15, wherein the migration detection agent comprises a detection agent for CD106 or a functional equivalent thereof.

17. A method for treating and/or preventing diabetes mellitus or a disease, disorder, and/or symptom associated with diabetes mellitus in a subject, the method comprising: administering an effective amount of an agent that reduces or eliminates an abnormal hematopoietic stem cell (HSC) and a stem cell migration agent to the subject.

18. A method for using a migration and/or residual state of an abnormal hematopoietic stem cell (HSC) as an indicator of treatment for treating and/or preventing diabetes mellitus or diabetes mellitus and/or a disease, disorder, and/or symptom associated with diabetes mellitus in a subject, the method comprising: detecting the migration and/or residual state of the abnormal hematopoietic stem cell (HSC) in the subject.
